(19)

Europäisches Patentamt
European Patent Office
Office européen des brevets

(11) **EP 3 491 996 A1**

(12) **EUROPEAN PATENT APPLICATION**
published in accordance with Art. 153(4) EPC

(43) Date of publication:
**05.06.2019 Bulletin 2019/23**

(21) Application number: **17834563.3**

(22) Date of filing: **28.07.2017**

(51) Int Cl.:
**A61B 3/10** (2006.01)

(86) International application number:
**PCT/JP2017/027547**

(87) International publication number:
**WO 2018/021561 (01.02.2018 Gazette 2018/05)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **29.07.2016 JP 2016150320**
**01.09.2016 JP 2016171311**
**30.09.2016 JP 2016194227**

(71) Applicant: **Nidek Co., Ltd.**
**Gamagori-shi, Aichi 443-0038 (JP)**

(72) Inventors:
• **ENDO, Masakazu**
**Gamagori-shi**
**Aichi 443-0038 (JP)**
• **HANEBUCHI, Masaaki**
**Gamagori-shi**
**Aichi 443-0038 (JP)**
• **HANAKI, Miwako**
**Gamagori-shi**
**Aichi 443-0038 (JP)**
• **ARIKAWA, Toru**
**Gamagori-shi**
**Aichi 443-0038 (JP)**

(74) Representative: **Hoefer & Partner Patentanwälte mbB**
**Pilgersheimer Straße 20**
**81543 München (DE)**

(54) **OPHTHALMOLOGIC DEVICE AND IOL POWER DETERMINATION PROGRAM**

(57) The present invention addresses the technical problem of providing: an ophthalmologic device that calculates an appropriate intraocular lens power; and an IOL power determination program. Provided is an ophthalmologic device for determining the power of an IOL to be inserted to an eye to be examined, wherein the ophthalmologic device is characterized in that: a parameter acquisition means for acquiring a plurality of eye shape parameters of the eye to be examined and a calculation control means for calculating the IOL power are provided; and the calculation control means inputs the plurality of eye shape parameters to a mathematic model trained by a machine learning algorithm, thereby causing IOL related information to be output from the mathematic model.

START

S1 — ALIGNMENT

S2 — CAPTURE FRONT IMAGE

S3 — CAPTURE TOMOGRAPHIC IMAGE

S4 — ACQUIRE EYE SHAPE PARAMETER

S5 — INPUT PARAMETER TO MATHEMATIC MODEL

S6 — OUTPUT PREDICTED POSTOPERATIVE ANTERIOR CHAMBER DEPTH

S7 — SUBSTITUTE EACH PARAMETER INTO EXPRESSION

S8 — ACQUIRE IOL POWER

END

Fig. 3

EP 3 491 996 A1

**Description**

**Technical Field**

The present disclosure relates to an ophthalmologic device and an IOL power determination program for determining a power of an IOL to be inserted in a subject eye.

**Background Art**

An ophthalmologic device that determines a power of an intraocular lens (IOL) to be inserted in a subject eye after a crystalline lens substance is removed in a cataract surgery has been known (see Patent Literature 1). In such a device, the IOL power is calculated by using, for example, a regression formula or a theoretical formula in order to determine the intraocular lens power.

In the device described in Patent Literature 1, a predicted postoperative anterior chamber depth (position of the intraocular lens) is estimated in order to determine the intraocular lens power.

An ophthalmologic device that captures an anterior segment of the subject eye in order to determine the power of the IOL has been described (see Patent Literature 2).

**Citation List**

**Patent Literature**

Patent Literature 1: JP-A-2011-206368
Patent Literature 2: JP-A-2013-094410

**Summary of Invention**

As a first problem, an appropriate IOL power may not be necessarily acquired in the calculation formula of the related art. For example, there are some cases where an error in calculation of the IOL power becomes large in a eye which is not an average shape.

As a second problem, variance in the flexible condition of a loop of the intraocular lens is not considered in the estimation of the predicted postoperative anterior chamber depth of the related art.

As a third problem, the intraocular lens may revolve around an optical axis after surgery in a case where the intraocular lens is inserted within a crystalline lens capsule of the subject eye, but the axis angle of a toric intraocular lens having an astigmatic component is changed in this case, and it is not possible to properly correct the astigmatism of the subject eye. Thus, it is necessary to investigate the relationship between the shape of the crystalline lens and the revolution of the intraocular lens in order to investigate the cause of the revolution of the intraocular lens, but it is not possible to easily acquire the shape of the crystalline lens in the ophthalmologic device of the related art.

The present disclosure has been made in view of the aforementioned problems, and a technical problem of the present disclosure is to provide an ophthalmologic device and an IOL power determination program which solve at least one of the problems of the related art.

In order to resolve the first problem, a first embodiment according to the present disclosure is characterized by having the following configurations.

(1) There is provided an ophthalmologic device for determining a power of an IOL to be inserted in a subject eye, including a parameter acquisition portion that acquires a plurality of eye shape parameters of the subject eye, and a calculation control portion that calculates the IOL power, in which the calculation control portion outputs IOL related information from a mathematic model trained by a machine learning algorithm after inputting the plurality of eye shape parameters into the mathematic model.

(2) There is provided an IOL power determination program executed in an ophthalmologic device for determining a power of the IOL to be inserted in a subject eye, in which the IOL power determination program is executed by a processor of the ophthalmologic device to cause the ophthalmologic device to perform a parameter acquisition step of acquiring a plurality of eye shape parameters of the subject eye, and a calculation step of outputting IOL related information from a mathematic model trained by a machine learning algorithm after inputting the plurality of eye shape parameters to the mathematic model.

In order to resolve the second problem, a second embodiment according to the present disclosure is characterized by having the following configurations.

(3) There is provided an ophthalmologic device for determining a power of an intraocular lens to be inserted in a subject eye, including a cross-sectional imaging portion that captures an anterior segment cross-sectional image of the subject eye, and a calculation control portion that calculates the power of the intraocular lens, in which the calculation control portion analyzes the anterior segment cross-sectional image to acquire a capsule diameter of a crystalline lens of the subject eye, estimates a predicted postoperative anterior chamber depth of the intraocular lens by using the capsule diameter, and calculates the power of the intraocular lens based on the predicted postoperative anterior chamber depth.

(4) There is provided an IOL power determination program executed in an ophthalmologic device that determines a power of an intraocular lens to be inserted in a subject eye, in which the IOL power determination program is executed by a processor of the ophthalmologic device to cause the ophthalmologic device to perform a cross-section imaging step of capturing an anterior segment cross-sectional image of the subject eye, a capsule diameter acquisition step of analyzing the anterior segment cross-sectional image captured in the cross-section imaging step to acquire a capsule diameter of a crystalline lens of the subject eye, an estimation step of estimating the predicted postoperative anterior chamber depth of the intraocular lens by using the capsule diameter acquired in the capsule diameter acquisition step, and a calculation step of calculating the power of the intraocular lens based on the predicted postoperative anterior chamber depth estimated in the estimation step.

In order to resolve the third problem, a third embodiment according to the present disclosure is characterized by having the following configurations.

(5) There is provided an ophthalmologic device for imaging an anterior segment of a subject eye, including a tomographic imaging portion that captures an anterior segment tomographic image of the subject eye, and a calculation control portion that calculates at least any position of a major axis and a minor axis of a crystalline lens of the subject eye based on the anterior segment tomographic image.

**Brief Description of Drawings**

Fig. 1 is a schematic configuration diagram for describing a configuration of an ophthalmologic device according to the present example.
Fig. 2 is a diagram showing an example of a mathematic model.
Fig. 3 is a diagram showing a flowchart of a control operation.
Fig. 4 is a diagram showing an anterior segment observation screen on which a captured anterior segment image is displayed.
Fig. 5 is a diagram showing an example of an anterior segment cross-sectional image.
Fig. 6 is a diagram for describing the calculation of a predicted ELP.
Fig. 7 is a diagram for describing the calculation of the predicted ELP.
Fig. 8A is a diagram for describing a correction amount.
Fig. 8B is a diagram for describing the correction amount.
Fig. 9 is a diagram for describing parameters of an IOL.
Fig. 10 is a diagram showing a scanning direction of an OCT optical system.
Fig. 11 is a diagram showing the relationship between an axis angle and a curvature radius of a crystalline lens.
Fig. 12 is a diagram showing a display screen of an eyeball model.
Fig. 13 is a diagram showing a display screen of a toric axis.
Fig. 14A is a diagram showing the scanning direction of the OCT optical system.
Fig. 14B is a diagram showing the scanning direction of the OCT optical system.

**Description of Embodiments**

<First Embodiment>

An ophthalmologic device according to a first embodiment will be described. The ophthalmologic device (for example, an ophthalmologic device 10) determines the power of an intraocular lens (also referred to as an IOL) to be inserted in a subject eye. For example, the present device mainly includes a parameter acquisition unit (for example, an OCT device 5, a control unit 80, or an operation unit 84) and a calculation control unit (for example, a control unit 80). For example, the parameter acquisition unit acquires a plurality of eye shape parameters of the subject eye. For example, an eye shape parameter may be a corneal shape parameter such as a corneal anterior surface curvature, a corneal posterior surface curvature, a corneal thickness, a corneal width, or a corneal height, may be a crystalline lens shape parameter such as a crystalline lens anterior surface curvature, a crystalline lens posterior surface curvature, a crystalline lens thickness, a crystalline lens diameter, or a capsule diameter, may be an overall shape parameter such as an eye axial length, an anterior chamber depth, a corner angle, a corner angle distance, or a pupil diameter, or may be a retinal shape parameter such as a retinal thickness. The curvature of the crystalline lens during contraction or the curvature of the crystalline lens during relaxation may be used, or both the curvatures thereof may be used. For example, the parameter acquisition unit acquires the eye shape parameter based on a tomographic image captured by an OCT optical system. The parameter acquisition unit may acquire the eye shape parameters of an examinee from a server outside the device via a communication network or may acquire the eye shape parameters by the input from the operation unit.

The crystalline lens shape parameters include the size or position of the major axis or minor axis in the case where the crystalline lens is ellipsoid.

The calculation control unit calculates IOL related information by using a machine learning algorithm. The machine learning algorithm is, for example, a neural network, random forest, boosting, or the like.

The neural network is a method of mimicking a behavior of a neuronal network of an organism. The neural network is, for example, a feedforward (forward propagation type) neural network, an RBF network (radial basis function), a spiking neural network, a convolutional neural network, a recursive neural network (a recurrent neural network or a feedback neural network), a probabilistic neural network (a Boltzmann machine or a Bayesian network), or the like.

The boosting is a method of generating a strong classifier by combining a plurality of weak classifiers. The simple and weak classifiers are sequentially learned, and the strong classifier is constructed.

The random forest is a method of generating multiple decision trees by performing learning based on randomly sampled training data. In a case where the random forest is used, branches of a plurality of decision trees learned as classifiers in advance are traced, and results acquired from the respective decision trees are averaged (or decided by majority).

For example, the calculation control unit acquires the IOL related information by using a mathematic model (for example, a mathematic model 800) trained by the machine learning algorithm. For example, the mathematic model refers to a data structure for predicting the relationship between input data and output data. The mathematic model is constructed by being trained using a training dataset. The training dataset is a set of input training data and output training data. The input training data is sample data to be input to the mathematic model. For example, the eye shape parameter measured in the past is used as the input training data. The output training data is sample data of a value to be predicted by the mathematic model. For example, a postoperative anterior chamber depth or an IOL power are used as the output training data. The postoperative anterior chamber depth is the distance from the cornea to the IOL. In a case where certain input training data is input, the mathematic model is trained such that the output training data corresponding to the certain input training data is output. For example, correlation data (for example, weight) between the inputs and the outputs is updated through training. The calculation control unit calculates the IOL related information based on the correlation data.

For example, the calculation control unit may acquire the output of a predicted postoperative anterior chamber depth as the IOL related information from the mathematic model. In this case, the trained mathematic model may be a mathematic model that outputs a predicted postoperative anterior chamber depth from the input of the eye shape parameters including at least any of the crystalline lens anterior surface curvature, the crystalline lens posterior surface curvature, the crystalline lens thickness, or the anterior chamber depth. For example, as the input for the mathematic model that outputs a predicted postoperative anterior chamber depth, the two parameters crystalline lens anterior surface curvature and crystalline lens posterior surface curvature may be used, the three parameters crystalline lens anterior surface curvature, the crystalline lens posterior surface curvature, and anterior chamber depth may be used, or the four parameters crystalline lens anterior surface curvature, crystalline lens posterior surface curvature, anterior chamber depth, and crystalline lens thickness may be used. At least parameters of both anterior segment front face shape (for example, anterior surface curvature) and anterior segment posterior surface shape (for example, posterior surface curvature) which are acquired by an anterior segment cross-section imaging device may be used as the input for the mathematic model. As the output of the mathematic model, an offset distance from the crystalline lens anterior surface to the contact point of the crystalline lens and the zonule of Zinn may be acquired as the IOL related information. The predicted postoperative anterior chamber depth may be acquired by adding the acquired offset distance to the anterior chamber depth. In this case, the crystalline lens thickness may be added as an input for the mathematic model.

For example, the calculation control unit may calculate the IOL power based on the predicted postoperative anterior chamber depth output by the mathematic model. For example, the calculation control unit obtains the IOL power by substituting the predicted postoperative anterior chamber depth and other eye shape parameters into an IOL power calculation formula. For example, the calculation control unit may calculate the IOL power by substituting the predicted postoperative anterior chamber depth calculated by the mathematic model into the corresponding part of the existing IOL power calculation formulas such as an SRK/T formula, a Binkhorst formula, a Holladay formula, a Holladay 2 formula, a Hoffer-Q formula, an Olsen formula, and a Haigis formula. The machine learning function is used in this manner, and thus, it is possible to calculate an appropriate IOL power for eyes being examined that have various shapes.

The calculation control unit may output the IOL power as the IOL related information from the mathematic model by inputting the plurality of eye shape parameters to the mathematic model. In this case, the trained mathematic model may be a mathematic model that outputs the IOL power after the input of the corneal curvature, the eye axial length, the predicted postoperative anterior chamber depth or the like. For example, as the input for the mathematic model that outputs the IOL power, the two parameters corneal curvature and eye axial length may be used, the two parameters corneal curvature and predicted postoperative anterior chamber depth may be used, the two parameters eye axial length and predicted postoperative anterior chamber depth may be used, or the three parameters corneal curvature, eye axial length, and predicted postoperative anterior chamber depth may be used. In this example, the corneal curvature may be the corneal anterior surface curvature, may be the corneal posterior surface curvature, or may be both thereof.

For example, the calculation control unit may output the IOL power from the mathematic model by calculating the predicted postoperative anterior chamber depth based on the plurality of eye shape parameters, inputting the eye shape parameters and the predicted postoperative anterior chamber depth. As stated above, the calculation control unit may output the IOL power from the mathematic model after inputting a primary parameter which is an actual measurement value and a secondary parameter calculated from an actual measurement value.

The calculation control unit may use two trained mathematic models. For example, a first mathematic model may be a mathematic model that outputs the predicted postoperative anterior chamber depth after inputting a plurality of eye shape parameters such as the crystalline lens anterior surface curvature, the crystalline lens posterior surface curvature, and the crystalline lens thickness. A second mathematic model may be a mathematic model that outputs the IOL power after inputting eye shape parameters such as the predicted postoperative anterior chamber depth, the corneal curvature, and the eye axial length. In this case, the calculation control unit may output the IOL power from the second mathematic model by inputting the plurality of eye shape parameters to the first mathematic model and inputting the predicted postoperative anterior chamber depth output from the first mathematic model and the eye shape parameters to the second mathematic model which is different from the first mathematic model. Accordingly, it is possible to accurately predict the postoperative anterior chamber depth and the IOL power.

The calculation control unit may input a feature parameter to the mathematic model in addition to the eye shape parameter. In this case, the trained mathematic model may be a mathematic model that outputs the predicted postoperative anterior chamber depth, the IOL power, or the like after the input of at least any of eye shape parameter or a feature parameter. A feature parameter is included in the input to the mathematic model, and thus, it is possible to output ELP, IOL power, or the like which is acquired by taking influences other than the eye shape into consideration.

For example, the feature parameter is a parameter regarding at least any feature of race, gender, and age of the patient (examinee) and the surgeon (examiner). Further, the feature parameter may be a refractive index such as the corneal refractive index, crystalline lens refractive index, vitreous body refractive index, or aqueous humor refractive index of the patient. The feature parameter may a desired parameter (or requested parameter) such as the refractive power, visual acuity, work viewing distance, or the number of focal points of the IOL after surgery desired by the patient or the surgeon. The feature parameter may include a lens constant to be used in each IOL power calculation formula. For example, the lens constant may be the A-constant in the SRK/T formula, the Surgeon factor (SF) in the Holladay formula, the IOL constant in the Haigis formula, or the like.

A feature parameter may be a parameter of a feature of the IOL model. For example, the feature parameter may be the entire length of the IOL, or a diameter, a shape, or a material of a support section. For example, the feature parameter may be a parameter obtained by calculating a function of the equatorial dimension (for example, a diameter) of a crystalline lens capsule. For example, the function of the diameter of the crystalline lens capsule may be a function yielding the forward-backward displacement amount of an IOL optical section with respect to the length of a loop (support section) of the IOL at the time of being fixed within the eye.

For example, in a case where the feature parameter related to the IOL model or the ELP of the capsule diameter etc. is used as the feature parameter, the mathematic model may be a mathematic model that outputs the ELP by inputting feature parameters regarding the IOL model or the capsule diameter, the corneal curvature, the anterior chamber depth, and the eye axial length.

A mathematic model that outputs the ELP after inputting the eye shape parameters may be prepared for each IOL model instead of inputting feature parameters regarding the IOL model or the like to the mathematic model.

A feature parameter may be used as a correction coefficient in addition to being used in machine learning, the IOL power or the predicted postoperative anterior chamber depth acquired in the course machine learning being corrected. For example, the calculation control unit may obtain an effective ELP appropriate for each IOL model by obtaining a predicted position of the ELP (for example, distance from the cornea to the contact point of the crystalline lens capsule and the IOL loop) assumed not to depend on the IOL model through the machine learning and correcting a temporary predicted ELP by using the feature parameter related to the IOL model or an ELP such as the capsule diameter. Accordingly, it is possible to reduce the complexity of the procedure of predicting an ELP value for each IOL model by using the machine learning.

The calculation control unit may output selection information for selecting an appropriate IOL power calculation formula among the SRK/T formula, the Binkhorst formula, the Holladay formula, the Holladay 2 formula, the Hoffer-Q formula, the Olsen formula, the Haigis formula, etc. as the mathematic model. In this case, the mathematic model may be a mathematic model that outputs information regarding the appropriate IOL calculation formula after the input of feature values required in the selection of the calculation formula. For example, the information may be information indicating the appropriate formula among a plurality of IOL power calculation formulas or may be a recommendation degree of each IOL power calculation formula. For example, the calculation control unit may output the information regarding selection of an IOL power calculation formula to be the mathematic model by inputting feature values related to the eye axial length, the IOL model, the surgeon, the race, the gender, and/or the age to the mathematic model.

The present device may further include a presentation unit (for example, a monitor 70) that presents the selection information output by the mathematic model to a user. The present device may further include a warning unit (for example, a monitor 70) that warns a user in a case where the user attempts to use an inappropriate formula in the selection information output by the mathematic model.

The calculation control unit may optimize the constants or the coefficients used in the IOL power calculation formula by the aforementioned mathematic model. Accordingly, it is possible to calculate the IOL power using appropriate values rather than a previously used values as the constants or the coefficients.

The calculation control unit may execute an IOL power determination program stored in a storage unit (for example, a memory 85) or the like. The IOL power determination program includes, for example, a parameter acquisition step and a calculation step. The parameter acquisition step is a step of acquiring the plurality of eye shape parameters of the subject eye. The calculation step is a step of outputting the IOL related information from the mathematic model after inputting the plurality of eye shape parameters to the mathematic model trained by the machine learning algorithm.

<Second Embodiment>

Hereinafter, a second embodiment according to the present disclosure will be described. For example, an ophthalmologic device (for example, an ophthalmologic device 10) according to the second embodiment includes a cross-section imaging unit (for example, an OCT device 5) and a calculation control unit (for example, a control unit 80). For example, the cross-section imaging unit captures an anterior segment cross-sectional image of the subject eye. For example, the cross-section imaging unit is an optical coherence tomography device (OCT), ultrasonic diagnostic equipment, a Scheimpflug camera, or the like.

The calculation control unit calculates the power of the intraocular lens (also referred to as an IOL) to be inserted in the subject eye. Initially, the calculation control unit acquires the capsule diameter of the crystalline lens by analyzing the anterior segment cross-sectional image. For example, the calculation control unit obtains an equatorial position which is the maximum diameter section of the crystalline lens from the anterior segment cross-sectional image, and obtains the diameter (capsule diameter) in this equatorial position. In a case where the equator of the crystalline lens is not able to be captured because it is hidden by the iris, the calculation control unit may obtain two points at which approximate curves of the crystalline lens anterior surface and the crystalline lens posterior surface cross each other, and may obtain a distance between these two points as the capsule diameter.

Subsequently, the calculation control unit estimates the postoperative anterior chamber depth of the intraocular lens from the acquired capsule diameter. The predicted postoperative anterior chamber depth is a value obtained by predicting a distance before surgery from the cornea to the optical section of the IOL. The predicted postoperative anterior chamber depth is used in the IOL power calculation, and influences the calculation result thereof. Since the postoperative anterior chamber depth is changed according to the flexibility of the support section (loop) of the IOL, the predicted postoperative anterior chamber depth is estimated by taking the flexibility of the loop into consideration. The flexibility of the loop is changed by the capsule diameter. For example, the flexibility of the loop becomes larger in a case where the capsule diameter is small compared to a case where the capsule diameter is large, in order that the IOL fits within the capsule. Accordingly, the calculation control unit corrects misalignment in predicted postoperative anterior chamber depth due to the flexibility of the loop, according to the size of the capsule diameter. For example, since the flexibility of the loop becomes large in a case where the capsule diameter is small compared to a case where the capsule diameter is large, the calculation control unit increases the correction amount of the predicted postoperative anterior chamber depth toward a posterior capsule or an anterior capsule. For example, the correction amount of the predicted postoperative anterior chamber depth, which is set as is customary according to the size of the capsule diameter, may be stored in the memory, and the correction amount may be read out from the memory by the calculation control unit at the time of predicting the postoperative anterior chamber depth. For example, the calculation control unit may correct the predicted postoperative anterior chamber depth toward the posterior capsule by 0.5 mm in a case where the capsule diameter is 10 mm, and may correct the predicted postoperative anterior chamber depth toward the posterior capsule by 1 mm in a case where the capsule diameter is 9 mm.

The calculation control unit may estimate the predicted postoperative anterior chamber depth by using feature parameters of the IOL in addition to the capsule diameter. The feature parameters (model information of the IOL are, for example, parameters determined by at least any of an entire length, a thickness, an elastic modulus, an optical section diameter, and a loop angle of the IOL. These parameters influence the flexible condition of the loop within the capsule. The calculation control unit estimates that the predicted postoperative anterior chamber depth is to be large in a case where the IOL has a feature parameter which makes it easy to bend. That is, the calculation control unit estimates that the loop is bent, and thus, the amount by which the optical section of the IOL moves toward the posterior capsule is large. For example, in a case where the entire length of the IOL is large, since the loop needs to be greatly bent in order to fit in the crystalline lens capsule, the calculation control unit increases the correction amount of the predicted postoperative anterior chamber depth toward the posterior capsule. In a case where the thickness, the elastic modulus, or the like of the IOL is large and rigidity of the IOL is high, since the loop is difficult to bend, the calculation control unit decreases the correction amount of the predicted postoperative anterior chamber depth toward the posterior capsule. Since the IOL model may be bent forwards depending on the design, in this case the calculation control unit performs correction such that the predicted postoperative anterior chamber depth is closer to the anterior capsule. That is, the calculation control unit may change direction (toward the anterior capsule or the posterior capsule) depending on the IOL model.

The calculation control unit may compare the capsule diameter with the entire length of the IOL. The calculation control unit may determine whether or not the IOL is appropriate for the subject eye based on the comparison result. For example, the calculation control unit may determine that the IOL is appropriate in a case where the entire length of the IOL is larger than the capsule diameter, and may determine that the IOL is not appropriate in a case where the entire length of the IOL is smaller than the capsule diameter. For example, the calculation control unit may calculate the power of the intraocular lens in a case where the IOL is appropriate for the subject eye, and may notify the examiner that the intraocular lens is changed to another model in a case where the IOL is not appropriate.

The calculation control unit may calculate the correction amount of the predicted postoperative anterior chamber depth by using the mathematic model trained by the machine learning algorithm. In this case, for example, the calculation control unit may output the correction amount after inputting the capsule diameter or the like to the mathematic model.

<Third Embodiment>

Hereinafter, a third embodiment according to the present disclosure will be described. An ophthalmologic device (for example, an ophthalmologic device 10) according to the third embodiment captures at least the anterior segment of the subject eye. For example, the ophthalmologic device includes a tomographic imaging unit (for example, an OCT device 5) and a calculation control unit (for example, a control unit 80). The tomographic imaging unit captures the anterior segment tomographic image of the subject eye. The anterior segment tomographic image is an image including at least a cross section of the crystalline lens.

The calculation control unit calculates the position of the major axis or the minor axis in a case where the crystalline lens of the subject eye is ellipsoid, based on the anterior segment tomographic image. The positions of the major axis or the minor axis are rotation angles where the left-right direction (horizontal direction) of the subject eye is 0° or are coordinates, with the part of the subject eye as the origin. For example, the calculation control unit may obtain the curvature radius of the anterior surface or the posterior surface of the crystalline lens by analyzing the anterior segment tomographic image, and may calculate the major axis or the minor axis of the crystalline lens from the curvature radius thereof.

As stated above, the present device can calculate the position of the major axis or the minor axis of the crystalline lens from the tomographic image of the eye to be examined, and can easily acquire shape information of the crystalline lens. For example, the present device can be used for determining an insertion position of the intraocular lens in cataract treatment.

The present device may include a display control unit (for example, a control unit 80). The display control unit may display the position of the major axis or the minor axis of the crystalline lens on a display unit (for example, a monitor 70). For example, the display control unit may display the position of the major axis or the minor axis of the crystalline lens as graphics such as a line or a point, or may display the angle or the distance as a numerical value. The present device can provide useful information for the insertion position of the intraocular lens in the cataract treatment to the examiner by displaying the position of the major axis or the minor axis of the crystalline lens.

The display control unit may display a position of an astigmatic axis (also referred to as a toric axis) of a toric intraocular lens together with the position of the major axis or the minor axis of the crystalline lens on the display unit. For example, the display control unit may display the position of the major axis or the minor axis of the crystalline lens and the position of the astigmatic axis of the toric intraocular lens on a front face image of the anterior segment as a line or the like. As stated above, the present device can provide information regarding a positional relationship between the major axis or the minor axis of the crystalline lens and the astigmatic axis of the toric intraocular lens. For example, the toric axis may be displayed as graphics such as a line or point or may be displayed as a numerical value. The position of the graphics such as a line or point may be manually determined by the examiner.

The display control unit may display the position of a loop of the toric intraocular lens together with the position of the major axis or the minor axis of the crystalline lens on the display unit. For example, the loop is a support section for supporting the intraocular lens within the capsule of the crystalline lens. For example, the display control unit may display the figure of the intraocular lens or may display the position of the loop as the line, the point, or the like. The display control unit may display the position of the loop as a numerical value such as an angle or coordinates. As stated above, the present device can provide information regarding a positional relationship between the major axis or the minor axis of the crystalline lens and the loop of the toric intraocular lens.

The calculation control unit may calculate the misalignment amount between the major axis or the minor axis of the crystalline lens and the loop. Accordingly, the present device can acquire information regarding a possibility of the revolution of the intraocular lens. For example, the calculation control unit may calculate an angle difference between the position of the loop and the position of the major axis (or the minor axis) of the crystalline lens in a case where the axis of the toric intraocular lens is disposed with respect to the astigmatic axis of the eye at an ideal axis angle (for example, an astigmatic axis angle based on the corneal shape). Since the relationship between the axis angle of the toric intraocular lens and a position of a tip of the loop is determined for each intraocular lens, the calculation control unit may calculate the misalignment amount between the major axis or the minor axis of the crystalline lens and the position of the loop based on the axis angle of the toric intraocular lens. The ideal axis angle of the toric intraocular lens may be an axis angle obtained by taking induction of astigmatism into consideration. For example, induction of astigmatism is an astigmatism caused by a change in corneal shape due to corneal incision during cataract surgery. For example, the axis angle is estimated taking induction of astigmatism into consideration based on the corneal shape measured before surgery or the incision position or incision amount of the cornea.

The present device may include a notification unit. For example, the notification unit may be a display unit, a speaker, a lamp, or the like. In a case where the notification unit is provided, the calculation control unit may control the notification unit according to the misalignment amount between the position of the major axis or the minor axis of the crystalline lens and the position of the loop. For example, the calculation control unit may cause the notification unit to notify that there is a possibility that the toric intraocular lens is to revolve in a case where the position misalignment amount between the major axis of the crystalline lens and the loop exceeds a predetermined amount. For example, the calculation control unit may cause the notification unit to notify that there is the possibility that the toric intraocular lens will revolve in a case where the position of the tip of the loop is in the position of the minor axis of the crystalline lens. Accordingly, it is possible to prompt the examiner to adjust the axis angle of the toric intraocular lens. For example, the calculation control unit may cause the notification unit to communicate the direction in which the toric intraocular lens revolves. For example, the calculation control unit may cause the notification unit to communicate the direction in which the loop moves toward the major axis from the minor axis of the crystalline lens. Accordingly, it is possible to predict the revolution direction of the toric intraocular lens.

The present device may further include a front imaging unit that captures the front face image of the subject eye. In this case, the display control unit may display the position of the major axis or the minor axis of the crystalline lens on the front face image in a superimposition manner. Accordingly, the present device can provide information regarding a positional relationship between a feature part (sclera's blood vessel) of the subject eye and the major axis or the minor axis of the crystalline lens. The front face image is an image at the time of viewing the subject eye from the front. A tomographic imaging unit such as the OCT optical system may also function as the front imaging unit. In this case, the front face image may be a front face image generated from a set of tomographic image data captured by the tomographic imaging unit.

The calculation control unit may calculate a revolution amount of the toric intraocular lens by using the mathematic model trained by the machine learning algorithm. In this case, for example, the calculation control unit may output the revolution amount of the toric intraocular lens by inputting the position of the major axis or the minor axis of the crystalline lens, the position of the support section of the toric intraocular lens, or the like to the mathematic model.

<First Example>

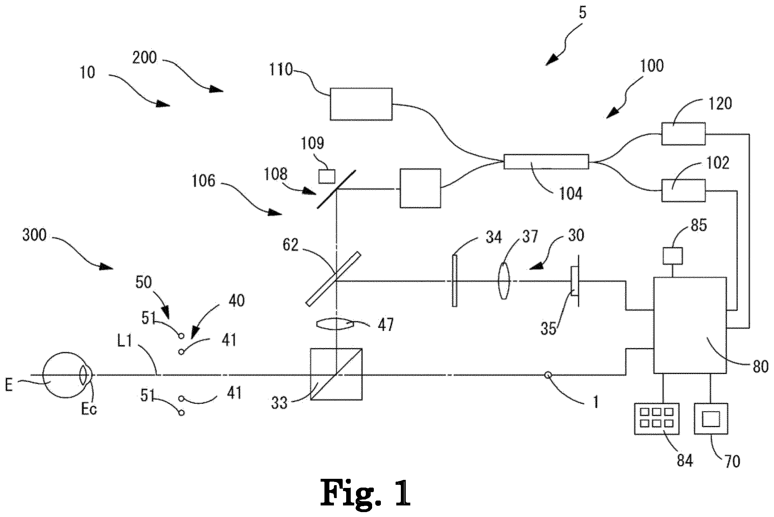

Hereinafter, the ophthalmologic device 10 according to the present disclosure will be described based on the drawings. Fig. 1 is a schematic configuration diagram showing a measurement unit 200 of the ophthalmologic device 10 according to the present example. The following optical system is built in a casing (not shown). The casing is three-dimensionally moved to a subject eye E through an operation controlling device (for example, a touch panel, a joystick, or the like) through the driving of a well-known alignment movement mechanism. In the following description, it is assumed that the optical axis direction of the subject eye (eye E) being examined is the Z direction, the horizontal direction is the X direction, and the vertical direction is the Y direction. A direction along the surface of the fundus may be considered as an XY direction.

In the following description, an example in which the ophthalmologic device 10 includes an optical coherence tomography device (OCT device) 5 and a corneal shape measurement device 300 will be described. The OCT device 5 is used as an anterior segment imaging device for capturing a cross-sectional image of the subject eye E. The OCT device 5 is used as an eye axial length measurement device for measuring an eye axial length of the eye E. The corneal shape measurement device 300 is used for measuring the corneal shape.

The OCT device 5 includes an interference optical system (OCT optical system) 100. The OCT optical system 100 irradiates the eye E with measurement light. The OCT optical system 100 detects an interference state between measurement light reflected from each part (for example, the cornea, the crystalline lens, the fundus, or the like) of the subject eye and reference light by using a light receiving element (detector 120). The OCT optical system 100 includes a scanning unit (for example, optical scanner) 108 that scans the measurement light on the subject eye in order to change the imaging position on the subject eye. The control unit 80 controls operation of the scanning unit 108 based on set imaging positional information, and acquires a cross-sectional image based on a light receiving signal from the detector 120.

The OCT optical system 100 has a device configuration of a so-called optical coherence tomography (OCT). The OCT optical system 100 splits light emitted from a measurement light source 102 into measurement light (sample light) and reference light by using a coupler (beam splitter) 104. The OCT optical system 100 guides the measurement light to the subject eye and guides the reference light to a reference optical system 110 by using the measurement optical system 106. Thereafter, interfered light arising from the combination of the measurement light reflected by each part of the subject eye and the reference light is received by the detector (light receiving element) 120.

The light emitted from the light source 102 is split into a beam of measurement light and a beam of reference light by using the coupler 104. The beam of measurement light passes through an optical fiber, and is then emitted to the air. The beam of measurement light is focused on the subject eye through the scanning unit 108 and another optical member of the measurement optical system 106. The light reflected by each part of the subject eye is returned to the optical fiber through the same optical path.

The scanning unit 108 scans the measurement light on the eye E in the XY direction (transversal direction). The scanning unit 108 is, for example, two galvanometer mirrors, and a reflection angle thereof is optionally adjusted by a driving mechanism 109.

Accordingly, the reflection (progression) direction of the beam of light emitted from the light source 102 is changed, and the beam of light is scanned in any direction on the eye E. Accordingly, the imaging position on the subject eye is changed. The scanning unit 108 may have a configuration for deflecting light. For example, an acousto-optic element (AOM) for changing the progress (deflection) direction of the light is used in addition to a reflection mirror (a galvanometer mirror, a polygon mirror, or a resonant scanner).

The reference optical system 110 generates the reference light being combined with the reflection light acquired by the reflection of the measurement light on the eye E. The reference optical system 110 may be a Michelson type or a Mach-Zehnder type. For example, the reference optical system 110 is formed of the reflection optical system (for example, the reference mirror), returns the light from the coupler 104 to the coupler 104 again by reflecting the light by using the reflection optical system, and guides the light to the detector 120. As another example, the reference optical system 110 is formed of a transmission optical system (for example, an optical fiber), and guides the light from the coupler 104 to the detector 120 by transmitting the light without returning the light to the coupler.

The reference optical system 110 has a configuration in which an optical path length difference between the measurement light and the reference light is changed by moving the optical member on the path of the reference light. For example, the reference mirror is moved in the optical axis direction. The configuration for changing the optical path length difference may be disposed on the path of the measurement light of the measurement optical system 106.

The detector 120 detects the interference state between the measurement light and the reference light. In a case where Fourier-domain OCT is used, spectral intensity of the interfered light is detected by the detector 120, and a depth profile (A-scan signal) in a predetermined range is acquired by Fourier transform for spectral intensity data. In this example, the control unit 80 can acquire the cross-sectional image by scanning the measurement light on each part of the subject eye by using the scanning unit 108. For example, the anterior segment cross-sectional image of the subject eye is captured. For example, the measurement light is scanned in the X direction or the Y direction, and thus, it is possible to acquire the cross-sectional image on an XZ plane or a YZ plane of the subject eye (in the present embodiment, a method of one-dimensionally scanning the measurement light on the anterior segment in this manner and acquiring the tomographic image is B-scan). The acquired cross-sectional image is stored in the memory 85 connected to the control unit 80. The measurement light is two-dimensionally scanned in the XY direction, and thus, it is possible to acquire a three-dimensional image of the subject eye.

For example, as the Fourier-domain OCT, there are spectral-domain OCT (SD-OCT) and swept-source OCT (SS-OCT). The Fourier-domain OCT also may be time-domain OCT (TD-OCT).

In a case where the SD-OCT is used, a low-coherent light source (broadband light source) is used as the light source 102, and a spectroscopic optical system (spectrometer) that splits the interfered light into frequency components (wavelength components) is provided in the detector 120. For example, the spectrometer includes a diffraction grating and a line sensor.

In a case where the SS-OCT is used, a wavelength scanning light source (wavelength tunable light source) for tem changing an emission wavelength at a high speed is used as the light source 102, and a single light receiving element is provided as the detector 120, for example. For example, the light source 102 is constituted by a light source, a fiber ring resonator, and a wavelength selection filter. For example, as the wavelength selection filter, there are a combination of the diffraction grating with the polygon mirror and a filter using a Fabry-Perot etalon.

The corneal shape measurement device 300 is roughly divided into a keratometer projection optical system 50, an alignment projection optical system 40, and an anterior segment front face imaging optical system 30.

The keratometer projection optical system 50 includes ringshaped light sources 51 disposed with a measurement optical axis L1 as a center, and is used for measuring the corneal shape (curvature, astigmatic axis angle, or the like) by projecting a ring index on the cornea of the subject eye. For example, an LED that emits infrared light or visible light is used as the light source 51. As the projection optical system 50, at least three or more point light sources may be disposed on the same circumference of a circle with the optical axis L1 as the center, or intermittent ring light sources may be used. A Placido index projection optical system that projects a plurality of ring indices may be used as the projection optical system.

The alignment projection optical system 40 is disposed inside the light source 51, includes projection light sources 41 (for example, λ = 970 nm) that emit infrared light, and is used for projecting an alignment index on a cornea Ec of the subject eye. The alignment index projected on the cornea Ec is used for performing position alignment (for example, automatic alignment, alignment detection, manual alignment, or the like) for the subject eye. In the present embodiment, the projection optical system 50 is an optical system that projects the ring index on the cornea Ec of the subject eye, and the ring index also functions as a Meyer ring. The light source 41 of the projection optical system 40 also functions as an anterior segment illumination that illuminates the anterior segment with the infrared light in an oblique direction. In the projection optical system 40, an optical system that projects parallel light on the cornea Ec may be provided, and the alignment in a forward and rearward direction may be performed by the combination with finite light using the projection optical system 40.

An observation optical system 30 is used for capturing (acquiring) the anterior segment front face image. The observation optical system 30 includes a dichroic mirror 33, an objective lens 47, a dichroic mirror 62, a filter 34, an imaging lens 37, and a two-dimensional imaging element 35, and is used for capturing the anterior segment front face image of the subject eye. The two-dimensional imaging element 35 is disposed in a position substantially conjugate with the anterior segment of the subject eye.

Anterior segment reflection light using the projection optical system 40 and the projection optical system 50 forms an image on the two-dimensional imaging element 35 through the dichroic mirror 33, the objective lens 47, the dichroic mirror 62, the filter 34, and the imaging lens 37.

A light source 1 is a fixation lamp. For example, a part of the anterior segment reflection light acquired from the anterior segment, of the light emitted from the light source 1, is reflected from the dichroic mirror 33, and forms an image by the anterior segment front face imaging optical system 30.

Hereinafter, a control system will be described. The control unit 80 performs the control of the entire device and the calculation of the measurement result. The control unit 80 is connected to the members of the OCT device 5, the members of the corneal shape measurement device 300, the monitor 70, the operation unit 84, the memory 85, and the like. In addition to various control programs, an IOL power calculation program or the like to be described below is stored in the memory 85.

Next, the IOL power calculation program to be used in the present example will be described. The mathematic model trained by the machine learning algorithm is used as the IOL power calculation program according to the present example. In the following description, an example in which a forward propagation type neural network is used will be described. The mathematic model in the neural network is generally constituted by an input layer for inputting data, an output layer for generating predicted data, and one or more hidden layers between the input layer and the output layer, and a plurality of nodes (also called units) is disposed in each layer. The node receives a plurality of inputs, and calculates one output. For example, data input to each node of each layer is output to each node of the adjacent layer. At this time, a different weight is added to each route. For example, an output value transmitted from a certain node to the next node is augmented or attenuated by the weight of each route. In a case where the data to which weight is added is input to the node, a function such as an activation function is applied to this data, and this data is output to each node of the next layer. This input is repeated between the adjacent layers, and the predicted data is ultimately output from the output layer.

For example, in a case where nodes i of a first layer are represented by 1, ..., I and nodes j of a second layer are represented by 1, ..., J, all values obtained by multiplying the inputs $x_i$ of the first layer by different weights $w_{ji}$ are added together, this value is added to one value $b_i$ called a bias, and this resultant value is the total input $u_j$ received by the nodes of the second layer as in the following Expression (1).

[Expression 1]

$$u_j = \sum_{I=1}^{I} w_{ji}x_i + b_i \qquad (1)$$

An output zi of the nodes of the second layer is an output of a function f such as an activation function for the total input ui as in the following Expression (2). For example, as the activation function, there are functions such as a logistic sigmoid function, a hyperbolic tangent function, a normalized linear function, and maxout.

[Expression 2]

$$z_i = f(u_j) \qquad (2)$$

The mathematic model in the neural network is trained by using the training dataset, and thus, it is possible to perform prediction regarding new data. For example, the training dataset is the set of input training data and output training data, and the weight of each node of each layer and the bias are adjusted such that a value close to the output training data is output from the output layer in a case where the input training data is input to the input layer. A plurality of training datasets is prepared, and the weight and the bias are repeatedly adjusted. Thus, it is possible to acquire the weight and the bias with versatility for various data, and it is possible to perform prediction for unknown data. For example, the training of the mathematic model is continued until an error between the output calculated from the input training data and the corresponding output training data falls within an allowed range. Back propagation (back propagation method) or the like is used in the adjustment of the weight.

In the trained mathematic model, the adjusted weight represents the correlation between the input and the output. The mathematic model outputs a predicted value calculated from an input of new data different from the training data based on this correlation. For example, each weight for the output of each node is applied as a coefficient. Accordingly, the correlation acquired through the training is reflected on the output of the mathematic model.

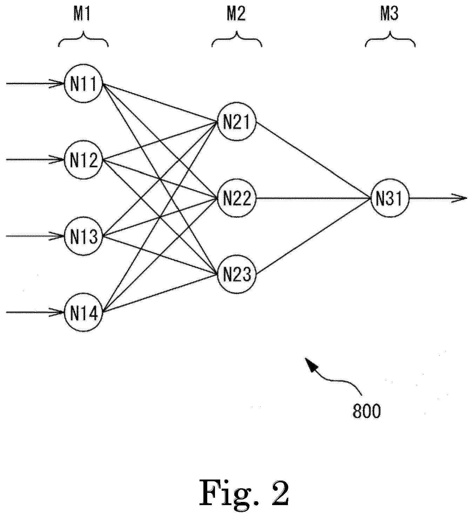

As shown in Fig. 2, in a mathematic model 800 according to the present example, four nodes N11 to N14 are disposed in an input layer M1, three nodes N21 to N23 are disposed in an intermediate layer M2, and one node N31 is disposed in an output layer M3. The mathematic model 800 is trained by using a plurality of training datasets using the crystalline lens anterior surface curvature, the crystalline lens posterior surface curvature, the crystalline lens thickness, and the anterior chamber depth of a certain examinee, and the postoperative anterior chamber depth (ELP) as one set. That is, in the mathematic model 800 according to the present example, in a case where four eye shape parameters of the crystalline lens anterior surface curvature, the crystalline lens posterior surface curvature, the crystalline lens thickness, and the anterior chamber depth are input to the input layer M1, the predicted postoperative anterior chamber depth (the predicted value of the postoperative anterior chamber depth) is generated so as to be output from the output layer M3. The mathematic model 800 is implemented by using any computer language or the like so as to be executed on a processor of the control unit 80.

<Control Operation>

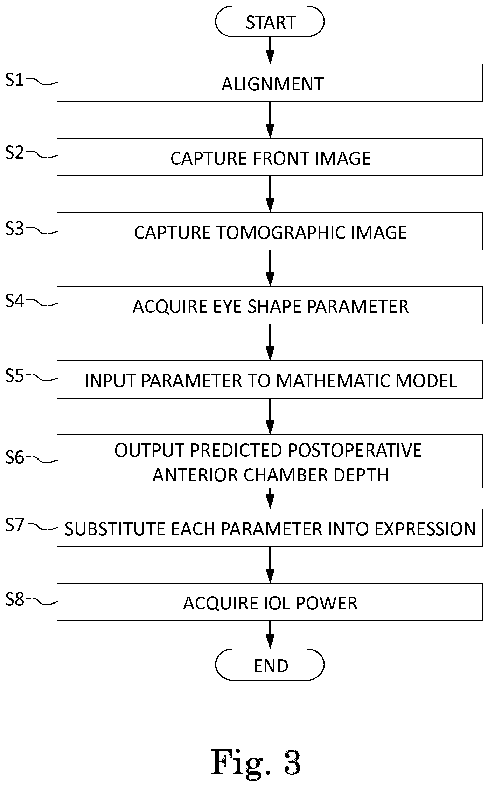

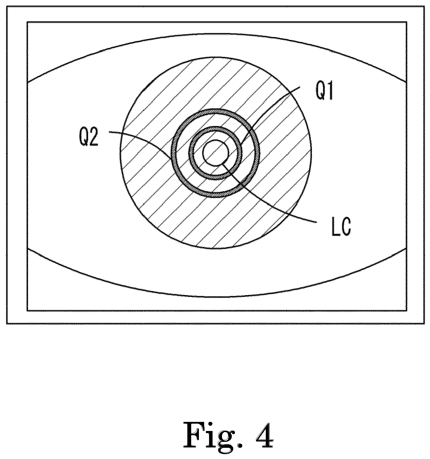

Hereinafter, a control operation of the present device will be described. Fig. 3 is a simplified flowchart of the control operation in a case where the intraocular lens power is determined by using the present device. In step S1, the control unit 80 performs the alignment of the measurement unit 200 for the subject eye. The control unit 80 turns on the light source 1, the light source 41, and the light source 51 at the time of the alignment. At this time, the front face image of the subject eye which is captured by the two-dimensional imaging element 35 is displayed on the monitor 70 as shown in Fig. 4. For example, an electrically displayed reticle LC, a ring index Q1 and a ring index Q2 formed by the light sources 41 and the light sources 51 are captured on the front face image.

In a case where the examiner causes the examinee to fix his or her gaze on the fixation lamp, the examiner operates the operation unit such that the touch panel such that the reticle LC and the ring index Q1 become concentric circles. The control unit 80 moves the measurement unit up, down, left, and right according to the operation received by the operation unit. Accordingly, the alignment is performed in the XY direction such that the optical axis L1 of the present device passes through a corneal vertex of the subject eye. The examiner operates the operation unit such that the ring index Q1 is in focus. The control unit 80 moves the measurement unit in the forward and rearward direction according to the operation received by the operation unit.

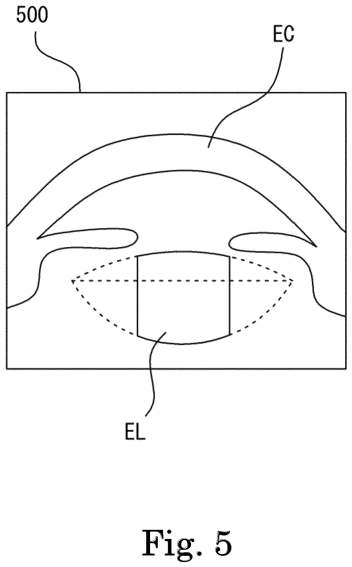

In a case where the alignment for the anterior segment is completed, the control unit 80 captures the front face image of the anterior segment of the subject eye by the observation optical system 30 (step S2). The control unit 80 captures a cross-sectional image 500 of the subject eye shown in Fig. 5 by using the OCT optical system 100 based on a preset scanning pattern (step 3). The acquired anterior segment image and cross-sectional image are stored in the memory 85 or the like.

Subsequently, the control unit 80 acquires the eye shape parameter in step 4. For example, the control unit 80 calculates the corneal shapes of the subject eye based on the ring index images Q1 and Q2 on an anterior segment image 400 stored in the memory 85. For example, the corneal shape specifically is the corneal curvature radius of the corneal anterior surface, astigmatic axis angle of the cornea, or the like in the steep meridian direction and flat meridian direction. The control unit 80 analyzes the cross-sectional image captured by using the OCT device 5. For example, the control unit 80 detects the position of the cornea, the crystalline lens, or the like through edge detection of the cross-sectional image, and measures the corneal thickness, the anterior chamber depth, or the crystalline lens thickness based on the position thereof. The control unit 80 performs circular approximation (or ellipsoid approximation, conic-curve approximation, or the like) on the detected anterior surfaces or posterior surfaces of the cornea and the crystalline lens, and measures the curvature radius of the corneal posterior surface, the crystalline lens anterior surface curvature, the crystalline lens posterior surface curvature, or the like based on this approximate curve. In a case where it is possible to capture the retina by using the OCT optical system, the control unit measures the eye axial length. For example, the acquired eye shape parameters are stored in the memory 85 or the like.

In step S5, the control unit 80 reads out the crystalline lens anterior surface curvature, the crystalline lens posterior surface curvature, the crystalline lens thickness, and the anterior chamber depth acquired by the measurement unit 200 from the memory 85, and inputs the acquired parameters to each node of the input layer M1. The control unit 80 performs the calculation according to the rules of the mathematic model 800, and outputs a value of the predicted postoperative anterior chamber depth from the output layer M3 (step S6). The control unit 80 stores the output predicted postoperative anterior chamber depth in the memory 85.

In a case where the acquisition of each parameter is completed, the control unit 80 calculates the intraocular lens power by partially using the known SRK/T formula, Binkhorst formula, or the like. For example, the control unit 80 acquires the IOL power by substituting the parameters into the SRK/T formula, the Binkhorst formula, or the like in step S7 (step S8). In a case where the SRK/T formula (the following Expression (3)) is used, the intraocular lens power is calculated from the corneal curvature radius, the eye axial length, the predicted postoperative anterior chamber depth, and the like.

[Expression 3]

$$IOL = \frac{1000 \times n_a \times (n_a \times R - n_c ml \times LO - 0.001 \times DR}{(LO - AD') \times (n_a \times R - n_c ml \times AD' - 0.001 \times DR}$$

$$\frac{\times (V \times (n_a \times R - n_c ml \times LO) + LO \times R))}{\times (V \times (n_a \times R - n_c ml \times AD') + AD' \times R))} \quad (3)$$

Where, R: corneal curvature radius [mm] (R = (nk - 1.000) x 1000/K), nk: refractive index selected by examiner, LO: AL + RT [mm], RT: thickness [mm] of retina (RT = 0.65696 - 0.02029 x AL), AL: eye axial length [mm], AD': correction value [mm] of predicted anterior chamber depth (AD' = H + OF, OF = AD - 3.336), AD: predicted postoperative anterior chamber depth [mm] (AD = 0.62467 x A - 68.747), A: A-constant, H: corneal height [mm] (H = R - (R x R - ((Cw x Cw) / 4)) 1 / 2) (here, H = R in a case where (R x R - ((Cw x Cw) / 4)) < 0), Cw: corneal width [mm], Cw = -5.41 + 0.58412 x LC + 0.098 x K, LC: correction value [mm] of eye axial length (LC = AL in a case where AL ≤ 24.2, LC = -3.446 + 1.716 x AL - 0.0237 x AL2 in a case where AL > 24.2), DR: refractive power [D] of correction lens desired after surgery, LP: power [D] of IOL being implanted, V: vertex distance, na: refractive index (= 1.336) of aqueous humor and vitreous body, nc: refractive index (= 1.333) of cornea, and ncml: nc - 1 (= 0.333).

In the related art, a regression formula or a theoretical formula is used for the prediction of the ELP, and prediction accuracy is obtained to some degree as long as an eyeball follows a certain rule. However, the shape is not able to cope with a distinctive eyeball (for example, an eye of which an eye axis is long but an anterior chamber depth is shallow, an eye of which an eye axis is short but an anterior chamber depth is deep, or the like), and thus, an error in power is suddenly caused. As stated above, the postoperative anterior chamber depth is predicted by using the correlation between the plurality of eye shape parameters in the mathematic model trained by the machine learning, and thus, it is possible to calculate an appropriate IOL power with a higher probability even for the distinctive eyeball different from an average shape.

Although it has been described in the aforementioned example that the predicted postoperative anterior chamber depth is output by the mathematic model, the present invention is not limited thereto. For example, the IOL power may be output. In this case, the mathematic model is trained by using the plurality of eye shape parameters such as the corneal curvature, the eye axial length, and the anterior chamber depth as the input training data and using the IOL power as the output training data. Accordingly, in a case where the eye shape parameters measured by the measurement unit 200 are input, the control unit 80 generates the mathematic model that outputs the IOL power. It is possible to directly calculate the IOL power from the measured eye shape parameters by using this mathematic model.

The control unit 80 may acquire the toric IOL power by using the mathematic model. In this case, the mathematic model is trained by using the plurality of eye shape parameters as the input training data and using the toric IOL power as the output training data. In a case where the toric IOL power is obtained, the eye shape parameter includes, for example, at least any of corneal anterior surface astigmatic power, corneal anterior surface astigmatic axis, corneal posterior surface astigmatic power, corneal posterior surface astigmatic axis, corneal thickness (CT), anterior chamber depth ACD, postoperative anterior chamber depth (ELP), surgically induced astigmatism (SIA), pupil diameter (PS), crystalline lens anterior surface astigmatic power, crystalline lens anterior surface astigmatic axis, crystalline lens posterior surface astigmatic power, crystalline lens posterior surface astigmatic axis, an incision position, incision width, incision angle, an auxiliary port position, an auxiliary port number, or auxiliary port width. The incision position, the incision width, and the incision angle are the position, width, and angle (a direction of incision) of an incision to be made on the cornea, corneal border, or sclera of the subject eye at the time of inserting the IOL. The auxiliary port is, for example, an incision opening (or a wound) for inserting an auxiliary tool in the eye at the time of inserting the IOL. For example, the astigmatic power of the toric IOL to be inserted in the subject eye is calculated based on information of the crystalline lens anterior surface astigmatism or the crystalline lens posterior surface astigmatism. Toric IOL related information such as a toric angle or rotatability of the IOL after surgery may be obtained in addition to the toric IOL power. In a case where the rotatability of the IOL is acquired, a diameter of the crystalline lens capsule, a minor axis and a major axis of the crystalline lens capsule, the entire length of the IOL, or the like may be used as the input data for the mathematic model.

The calculation control unit may output the surgically induced astigmatism from a mathematic model after inputting feature parameters regarding at least any of incision position, the incision width, the incision angle, the auxiliary port position, the auxiliary port number, the auxiliary port width, and the surgeon to the mathematic model. The surgically induced astigmatism is astigmatism caused by the incision during cataract surgery. Accordingly, there are some cases where the surgically induced astigmatism is influenced by the features of the surgery of the surgeon. Thus, feature parameters regarding at least any of incision position, the incision width, the incision angle, the auxiliary port position, the auxiliary port number, the auxiliary port width, and the surgeon are used as input to the mathematic model in order to output the surgically induced astigmatism.

Although it has been described in the aforementioned example that the IOL power is obtained by substituting the predicted postoperative anterior chamber depth calculated by the mathematic model into the substitution part of the IOL power calculation formula, the IOL power may be calculated again by another mathematic model. For example, the IOL power may be acquired by using the mathematic model trained such that the IOL power is output by using the predicted postoperative anterior chamber depth calculated by the mathematic model and other eye shape parameters.

Although it has been described in the aforementioned example that there is one output of the neural network, a plurality of nodes is provided in the output layer, and a plurality of values may be output. For example, in a case where the eye shape parameters such as the corneal curvature, the eye axial length, the anterior chamber depth, the crystalline lens anterior surface curvature, or the crystalline lens posterior surface curvature are input, the two predicted postoperative parameters anterior chamber depth and the IOL power may be calculated. In this case, the mathematic model may be trained by using the plurality of eye shape parameters as the input training data and using the postoperative anterior chamber depth and the IOL power as the output training data. Of course, any number of nodes may be provided in the input layer and the hidden layer in addition to the output layer.

Although it has been described in the aforementioned example that the hidden layer uses one neural network, the hidden layer may use two or more deep neural networks. Similarly, in this case, the plurality of eye shape parameters may be input to each node of the input layer, and the predicted postoperative anterior chamber depth or the IOL power is ultimately output from the output layer via the plurality of hidden layers. IOL power information which reflects the correlation of each eye shape parameter in more detail may be acquired by using the deep neural networks.

Although it has been described in the aforementioned example that the predicted postoperative anterior chamber depth is calculated by regression using the neural network, the IOL related information may be acquired by classification using the machine learning. For example, the control unit 80 may select an appropriate IOL power calculation formula among the plurality of IOL power calculation formulas by using the mathematic model. For example, the control unit 80 acquires the appropriate IOL power by inputting the eye shape parameters to the mathematic model. Accordingly, it is possible to calculate the IOL power by using the IOL power calculation formula appropriate for the shape of the subject eye. In a case where the IOL power calculation formula is selected, feature parameters regarding the IOL model, the surgeon, the race, the gender, the age, or the like may be input to the mathematic model in addition to the eye shape parameters.

Although it has been described in the aforementioned example that the IOL power is calculated by the neural network, the present invention is not limited thereto. For example, another machine learning algorithm such as random forest or boosting may be used. For example, in a case where the random forest is used, IOL powers are obtained by several decision trees, and the IOL power is acquired by averaging the value of the IOL powers acquired by the decision trees. Which IOL power calculation formula is appropriate for the subject eye may be classified by the classifier acquired by the boosting. The network is not limited to the neural network, and it is possible to calculate an accurate IOL power by using the machine learning algorithm.

Although it has been described in the aforementioned example that the eye shape parameters to be input to the mathematic model are measured by the measurement unit 200, the eye shape parameters may be acquired from a server or the like. For example, a plurality of measurement results captured by a plurality of models of devices may be stored in the server via a network, and each device may acquire the data measured by another device from the server. For example, the control unit 80 may acquire the eye shape parameters from an electronic medical record system that manages registration information and measurement image data of the examinee.

Although it has been described in the aforementioned example that the IOL power is acquired by applying the predicted postoperative anterior chamber depth calculated by the trained mathematic model to the IOL power calculation formula, the IOL power may be output by inputting the predicted postoperative anterior chamber depth calculated by the theoretical formula and other eye shape parameters to the mathematic model. The calculation method of the predicted postoperative anterior chamber depth will be described below.

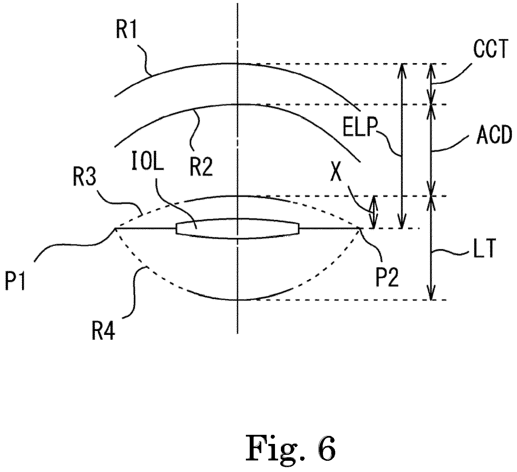

The calculation of the predicted postoperative anterior chamber depth using the theoretical formula will be described with reference to Fig. 6. The predicted postoperative anterior chamber depth is calculated by adding an offset amount X and a correction amount $\alpha$ (for example, a function of A-constant) to the corneal height (the height from the corneal anterior surface to the crystalline lens anterior surface). The corneal height is calculated by adding the corneal thickness CCT and the anterior chamber depth ACD.

The offset amount X represents the distance from the position of the crystalline lens anterior surface to the equatorial position (the maximum diameter section of the crystalline lens). The offset amount X does not take into consideration the movement amount (the correction amount $\alpha$ represented above) of the IOL toward the crystalline lens posterior capsule which is caused by a pressure applied to the IOL from the crystalline lens capsule, and excludes this movement amount (the details will be described below). The position of the tip of the support section (loop) of the IOL is substantially the same as the equatorial position. The equatorial position may be a position in which the crystalline lens anterior surface and the crystalline lens posterior surface cross each other.

Hereinafter, a method of calculating the offset amount X will be described. For example, the control unit 80 calculates the equatorial position by using a crystalline lens anterior surface curvature radius R3, a crystalline lens posterior surface curvature radius R4, and a crystalline lens thickness LT, and sets this equatorial position as the position of the tip of the loop after surgery. Since the position of the tip of the loop after surgery is substantially the same as the position of the optical section of the IOL, the offset amount X is calculated by using the position of the tip of the loop as the position of the optical section.

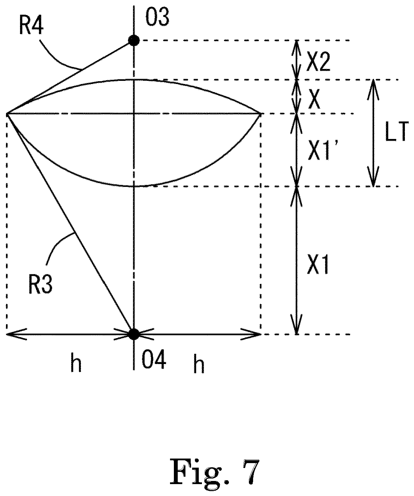

In Fig. 7, each of distances h represents a distance from the optical axis L1 to an intersection of an approximate circle of the crystalline lens anterior surface and an approximate circle of the crystalline lens posterior surface. A distance X1 represents a distance from a crystalline lens anterior surface curvature center O4 to the crystalline lens posterior surface at the optical axis L1. A distance X1' represents a distance from the intersection of the approximate circle of the crystalline lens anterior surface and the approximate circle of the crystalline lens posterior surface. A distance X2 represents a distance from a crystalline lens posterior surface curvature center O3 to a crystalline lens anterior surface at the optical axis L1. A distance X represents a distance from the intersection of the approximate circle of the crystalline lens anterior surface and the approximate circle of the crystalline lens posterior surface to the crystalline lens anterior surface, and is an offset amount. The following expression is established by the Pythagorean theorem.

[Expression 4]

$$h = \sqrt{{R_3}^2 - (X1 + X1')^2} = \sqrt{{R_3}^2 - (R_3 - X)^2}$$
$$h = \sqrt{{R_4}^2 - (X2 + X)^2} = \sqrt{{R_4}^2 - (R_4 - LT + X)^2} \tag{4}$$

Since the distances h are equal to each other in the following Expression (4), in the case where these expressions are solved for the offset amount X, the following expression is established.

[Expression 5]

$$X = \frac{2R_4 LT - LT^2}{2(R_3 + R_4 - LT)} \qquad (5)$$

The control unit 80 calculates the predicted postoperative anterior chamber depth based on the offset amount X calculated by Expression (5), the anterior chamber depth ACD, the corneal thickness CCT, and the correction amount $\alpha$. The correction amount $\alpha$ is a parameter for correcting the amount by which the IOL moves at the time of pushing the IOL toward the posterior capsule by a pressure of the capsule of the crystalline lens after the IOL is inserted. For example, the correction amount $\alpha$ varies depending on the model of the IOL, and is calculated by using the A-constant set for each model based on clinical data. The predicted postoperative anterior chamber depth ELP can be obtained by the following Expression (6).
[Expression 6]

$$ELP = CCT + ACD + X + \alpha \qquad (6)$$

There are some cases where the predicted postoperative anterior chamber depth is defined from the corneal posterior surface. In this example, the predicted postoperative anterior chamber depth is a distance from the corneal anterior surface to the IOL anterior surface.

The control unit 80 may directly calculate the IOL power by inputting the predicted postoperative anterior chamber depth calculated by Expression (6) and each eye shape parameter acquired by the measurement unit 200 to the mathematic model. As stated above, the eye shape parameter which is the actual measurement value (primary parameter) and the predicted postoperative anterior chamber depth which is the calculation value (secondary parameter) calculated based on the primary parameter may be used as the input of the mathematic model. Accordingly, the control unit 80 can calculate the IOL power which reflects the correlation between the primary parameter and the secondary parameter.

Eye diseases may be predicted by using the mathematic model trained by machine learning. For example, the control unit 80 may output a degree of progress of opacity of an intermediate transparent ocular part such as the cornea or the crystalline lens, a degree of progress of corneal degeneration such as keratoconus, post-LASIK corneal ectasia, percussive corneal marginal degeneration, postoperative disease incidence, or the like by inputting the eye shape parameters to the mathematic model. In a case where the degree of progress of corneal degeneration is acquired, the corneal curvature, the corneal thickness, or the like are used as the input for the mathematic model. In a case where the degree of progress of opacity of an intermediate transparent ocular part is acquired, a luminance value of the intermediate transparent part or the like to be captured on the anterior segment cross-sectional image is used as the input of the mathematic model. The mathematic model may include the plurality of nodes in the output layer, and may output the aforementioned disease information in addition to the IOL related information. Accordingly, the mathematic model can obtain the IOL power and acquire information regarding the state of the subject eye.

Although the IOL fixed within the capsule has been described in the aforementioned example, the present invention is not limited thereto. For example, the IOL related information can be acquired by using the mathematic model trained by the machine learning even in a case where the IOL for a phakic eye is inserted, a case where piggyback (two IOLs are inserted in the eye to which the IOL is inserted, or a case where the IOL is replaced. In a case where the piggyback or the replacement of the IOL is performed, the information regarding the IOL already inserted in the subject eye may also be used as one of the feature parameters for the input of the mathematic model.

The present disclosure is also applicable to a case where a three-dimensional shape image is acquired by acquiring the anterior segment tomographic image from a plurality of scanning positions in an optical coherence tomography device for capturing the anterior segment tomographic image (cross-sectional image). The OCT device 5 is the anterior segment imaging device that acquires the three-dimensional cross-sectional image of the anterior segment (three-dimensional anterior segment data), and the control unit 80 three-dimensionally obtains the offset distance from the crystalline lens anterior surface to the contact point of the crystalline lens and the zonule of Zinn based on the three-dimensional cross-sectional image acquired by the anterior segment imaging device. In this case, an average of the crystalline lens anterior surface curvature and the crystalline lens posterior surface curvature in each meridian direction in the three-dimensional anterior segment data is calculated, and the ELP is calculated based on the average. The accuracy of the acquired measurement value is improved by acquiring the measurement value from the three-dimensional shape image.

Although it has been described in the present example that the optical coherence tomography device for capturing the anterior segment tomographic image (cross-sectional image) is used as an example of the anterior segment imaging device that captures the anterior segment cross-sectional image, the present invention is not limited thereto. A projection optical system which projects emission light from a light source toward the anterior segment of the subject eye and forms a light cut section on the anterior segment and a light receiving optical system including a detector which receives light including anterior segment scattered light acquired through scattering in the anterior segment of the light cut section may be provided, and the anterior segment cross-sectional image may be formed based on a detected signal from the detector. That is, the present example is also applicable to a device or the like which projects slit light on the anterior segment of the subject eye and acquires an anterior segment cross-sectional image by a Scheimpflug camera.

The present invention is also applicable to a device which acquires a three-dimensional shape image of the anterior segment by rotating the Scheimpflug camera or moving the Scheimpflug camera in a horizontal or vertical direction. In this case, it is possible to accurately acquire the three-dimensional shape image of the anterior segment by performing misalignment correction at each predetermined rotation angle, and thus, the accuracy of the measurement value acquired from the three-dimensional shape image is improved. In this case, a position misalignment in a direction perpendicular to an imaging surface (slit section) is detected, and a misalignment correction process is performed based on the detection result.

Although the anterior segment cross-sectional image is optically acquired in the aforementioned configuration, the present invention is not limited thereto. For example, the anterior segment cross-sectional image may be acquired by detecting reflection information from the anterior segment by using an ultrasonic probe for B-scan.

Although it has been described in the present example that the IOL power calculation formula which is a regression formula or a theoretical formula such as the well-known SRK/T formula or the Binkhors formula is used as the calculation method of the IOL power, the present invention is not limited thereto. For example, it is possible to calculate the IOL power by a ray tracing method of simulating light behavior by geometrically tracing a reflection or refraction state of light. In this case, the IOL power is calculated by the ray tracing method by using the predicted postoperative anterior chamber depth ELP, the corneal thickness CCT, the eye axial length measurement result AL, the corneal curvature radius of the corneal anterior surface, and the corneal curvature radius of the corneal posterior surface. Since the ray tracing method is to calculate the IOL power by simulating the reflection or refraction of the light, it is possible to accurately calculate the IOL power by the IOL power calculation formula which is the theoretical formula.

Although it has been described in the present example that the corneal curvature radius on the corneal anterior surface is calculated by using the corneal shape measurement device 300 and the corneal curvature radius on the corneal posterior surface is calculated by using the OCT device 5, the present invention is not limited thereto. The corneal curvature radius on the corneal anterior surface may be calculated by the OCT device 5. The same measurement value may be used as the corneal curvature radius of the corneal anterior surface. That is, the corneal curvature radius on the corneal anterior surface calculated in the corneal shape measurement device 300 is used as the corneal curvature radius on the corneal anterior surface.

In the present example, corneal topography can be used as the corneal shape measurement device 300. In this case, since the curvature radius of the corneal anterior surface is calculated from the entire shape of the cornea at the time of calculating the curvature radius of the corneal anterior surface, the curvature radius of the corneal anterior surface is accurately calculated. Thus, the accuracy of the IOL power calculation is improved at the time of calculating the IOL power.

The present example is not limited to the device described in the present embodiment. For example, the IOL power calculation program that performs the function of the aforementioned example is supplied to the system or the device through a network or various storage media. A computer (for example, a CPU or the like) of the system or the device can read out the program, and can execute the readout program.

<Second Example>

Hereinafter, a second example will be described. An ophthalmologic device 10 according to the second example calculates a correction amount $\alpha$ of Expression (6) by using the capsule diameter. The configuration of the device is the same as the configuration of the first example, and thus, the description thereof will be omitted.

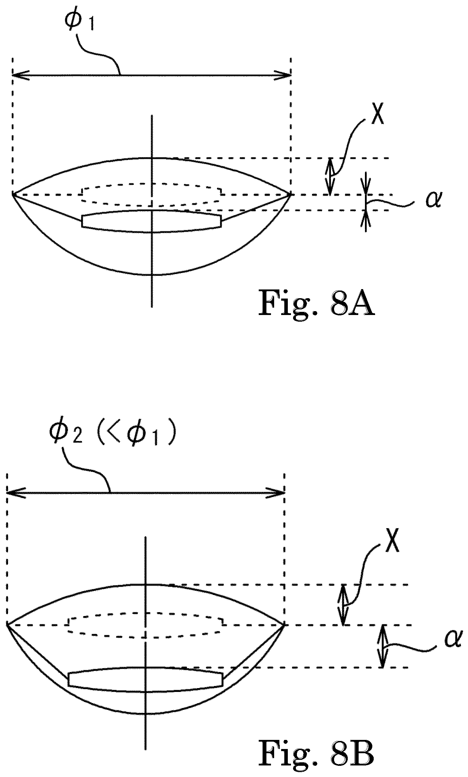

Since the movement amount varies depending on the model of the IOL, the correction amount $\alpha$ is calculated by using the A-constant set for each model based on clinical data. However, there are some cases where the flexible condition of the loop within the capsule is changed by the size of the capsule. For example, Fig. 8A shows a case where the size thereof is a capsule diameter $\varphi1$, and Fig. 8B shows a case where the size thereof is a capsule diameter $\varphi2$ smaller than the capsule diameter $\varphi1$. Since a pressure applied to the loop from the capsule in a case where the size thereof is the capsule diameter $\varphi2$ is larger than a pressure in a case where the size thereof is the capsule diameter $\varphi1$, the loop is greatly flexible toward the posterior capsule, and the correction amount $\alpha$ becomes large. As stated above, since the flexible condition of the loop varies depending on the magnitude of the capsule diameter, the correction amount $\alpha$ is calculated by taking the capsule diameter of the subject eye into consideration in the present example.

<Calculation of Correction Amount>

Hereinafter, the calculation of the correction amount α will be described. Initially, the control unit 80 measures the capsule diameter of the crystalline lens from the anterior segment cross-sectional image captured by the OCT device 5. For example, the control unit 80 performs approximation on the anterior surface and the posterior surface of the crystalline lens with a circle or an ellipsoid on the anterior segment cross-sectional image, and sets a distance between two intersections thereof as the capsule diameter. Since the capsule diameter is twice the distance h in Fig. 7, the control unit 80 may calculate the capsule diameter by Expression (4).

In a case where the capsule diameter is measured, the control unit 80 calculates the correction amount α by using the capsule diameter. For example, the control unit 80 calculates the correction amount α by Expression (7).
[Expression 7]

$$\alpha = (L/\phi) \times B + C \qquad (7)$$

Where, α: correction amount, L: entire length of IOL, φ: capsule diameter, B and C: feature parameter of each IOL.

For example, the correction amount α becomes large as the capsule diameter becomes small in Expression (7). In a case where the correction amount α becomes large, the predicted postoperative anterior chamber depth calculated by Expression (6) also becomes large. That is, the control unit 80 calculates the predicted postoperative anterior chamber depth as a large value in a case where the capsule diameter is small, and calculates the predicted postoperative anterior chamber depth as a small value in a case where the capsule diameter is large. As stated above, the control unit 80 corrects a misalignment in the predicted postoperative anterior chamber depth due to the flexible condition of the loop by changing the correction amount α depending on the capsule diameter.

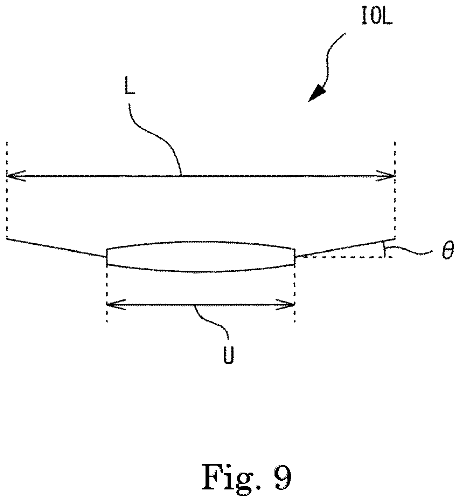

The control unit 80 may calculate the correction amount α by using the feature parameter of the IOL in addition to the capsule diameter of the subject eye. For example, as the feature parameter of the IOL, there are the entire length L, an optical section diameter U, a loop angle θ, and the like, as shown in Fig. 9. The feature parameter may be a parameter determined based on a thickness of the IOL, an optical section material, a loop material, a loop shape, or the like. Similarly to the capsule diameter, the feature parameters influence the flexible condition of the loop of the IOL.

For example, the loop tends to be more flexible as the entire length of the IOL becomes large. Accordingly, the control unit 80 calculates the correction amount α as a large value in a case where the entire length of the IOL is large. As stated above, the control unit 80 calculates the correction amount α as a large value as the loop of the IOL becomes more flexible. For example, since the loop tends to be more flexible as the rigidity of the IOL or the elastic modulus of the material becomes low, the correction amount α is calculated as a large value. These feature parameters may be measured for each model, and may be stored in the memory. Of course, values described in a catalog may be used.

The predicted postoperative anterior chamber depth estimated as stated above is substituted into AD' of, for example, Expression (3), and the intraocular lens power is calculated.

As stated above, the ophthalmologic device according to the second example can accurately calculate the predicted postoperative anterior chamber depth by taking a change in flexibility of the IOL due to the capsule diameter. Accordingly, it is possible to accurately calculate the power of the IOL for a distinctive eyeball which is not a standard eyeball shape.

The control unit 80 may compare the entire length of the IOL with the capsule diameter, and may change the control operation of the device depending on the comparison result. For example, the control unit 80 may change the control operation of the device in three cases of a case where the capsule diameter is smaller than the entire length of the IOL, a case where the capsule diameter is equal to the entire length of the IOL, and a case where the capsule diameter is larger than the entire length of the IOL.

For example, in a case where the capsule diameter is larger than the entire length of the IOL, the IOL revolves within the capsule, and the position of the IOL is not stable. In this case, there is a possibility that the IOL is not to be positioned in the predicted postoperative anterior chamber depth, and there is a concern that an error of the calculation result of the intraocular lens power is to be large. Accordingly, in a case where it is determined that the capsule diameter is larger than the entire length of the IOL, the control unit 80 may notify that the model of the IOL is not appropriate for the subject eye. For example, the control unit 80 may notify that the IOL larger than the entire length is changed, may notify that there is a possibility that a divergence between the refractive value measured after surgery and a predicted postoperative refractive value is to be large, or may notify that there is a possibility that the IOL is to revolve. The control unit 80 may notify that a load applied to the zonule of Zinn is to be large at the time of the insertion, and may predict a necessity of inserting a crystalline lens capsular tension ring (CTR). For example, the control unit 80 may display the aforementioned notification on the monitor 70 or may output the aforementioned notification with voice.

In a case where the entire length of the IOL is equal to the capsule diameter, the capsule holds the IOL, and the position of the IOL is stable. In this case, the IOL is positioned in the predicted postoperative anterior chamber depth. Accordingly, the control unit 80 may assume that the loop is hardly flexible, and may calculate the predicted postoperative anterior chamber depth while maintaining $\alpha = 0$.

In a case where the capsule diameter is smaller than the entire length of the IOL, the IOL is held by the capsule, and the position of the IOL is stable. The loop bends, and the optical section is stable in a position closer to the posterior capsule or the anterior capsule than a predicted position. Accordingly, the control unit 80 calculates the predicted postoperative anterior chamber depth by using the correction amount $\alpha$ calculated by Expression (7) described above by taking the flexibility of the IOL loop into consideration.

As stated above, it is possible to estimate an appropriate predicted postoperative anterior chamber depth by changing the control operation depending on the comparison result of the entire length of the IOL with the capsule diameter.

The correction amount $\alpha$ may be clinically (experimentally) set without using the aforementioned calculation formula. For example, the correction amount $\alpha$ may be set based on the relationship between the capsule diameter measured before surgery and the misalignment amount between the anterior chamber depth measured after surgery and the predicted postoperative anterior chamber depth.

The entire circumferential length (a length around the posterior capsule from the anterior capsule) of the crystalline lens capsule may be used instead of the crystalline lens capsule diameter. A total arc length by which the approximate circle of the crystalline lens anterior surface and the approximate circle of the crystalline lens posterior surface are cut from each other may be used as the entire circumferential length. It is possible to estimate the predicted postoperative anterior chamber depth acquired by taking the size of the crystalline lens into consideration by using this entire circumferential length in calculating the predicted postoperative anterior chamber depth.

Although it has been described in the aforementioned example that a design value is used as the feature parameter of the IOL, a value measured in vivo (in a living organism) may be used, a value measured in vitoro (in a test tube) may be used, or a value measured in the air may be used. In a case where the feature parameter is measured in the living organism, the feature parameter is measured by using an ultrasonic measuring device or the like.

In a case where the capsule diameter is acquired from the tomographic image, the control unit 80 may measure the capsule diameter in a position in which the loop of the IOL is in contact within the capsule. For example, in a case where the loop of the IOL is inserted in the crystalline lens so as to be in a direction of 90°, the control unit 80 may measure the capsule diameter by controlling the OCT optical system to perform scanning in the Y direction (90° direction) and analyzing the tomographic image in an upper and lower direction acquired in this manner. The position in which the loop of the IOL is in contact may be input by the examiner, and may be set by the control unit 80 based on the position of the major axis or the minor axis in a case where the major axis or the minor axis of the crystalline lens from the three-dimensional tomographic image data acquired by the OCT optical system 100 or the like is known.

<Third Example>

Hereinafter, a third example will be described. An ophthalmologic device 10 according to the third example can calculate the position of the major axis or the minor axis of the crystalline lens from the tomographic image of the subject eye, and can easily acquire shape information of the crystalline lens. For example, the present device can be used for determining an insertion position of the intraocular lens in cataract treatment. The configuration of the device is the same as the configuration of the first example, and thus, the description thereof will be omitted.

<Control Operation>

In a case where the alignment for the anterior segment is completed, the control unit 80 captures the anterior segment of the subject eye by the anterior segment front face imaging optical system 30. The captured anterior segment image is stored in the memory 85 or the like.

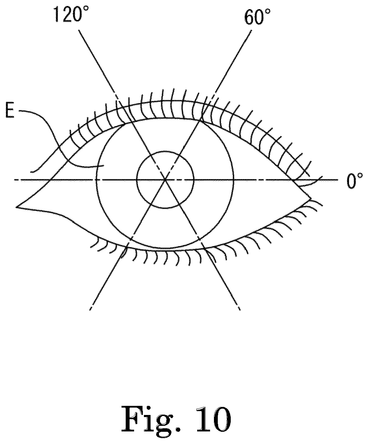

Subsequently, the control unit 80 captures tomographic images as a plurality of cross sections of the subject eye by the OCT optical system 100 based on a preset scanning pattern. For example, the control unit 80 radially performs scanning at any angle with an alignment center as a rotation axis, and captures at least three or more cross sections, as shown in Fig. 10. In the present example, the horizontal direction of the subject eye is set as 0°, and scanning is radially performed at angles of 0°, 60°, and 120°. It is possible to acquire data to be used in the estimation of the crystalline lens shape by a small number of times of scanning by radially performing the scanning. The acquired tomographic images are stored in the memory 85 or the like.

Since an eyeball optical axis (crystalline lens optical axis) is generally inclined with respect to a visual axis, an alignment reference may be adjusted so as to pass through the center of the crystalline lens. For example, the subject eye tilts by changing the position of the fixation lamp 1, and thus, the optical axis of the crystalline lens may be aligned with the optical axis L1 of the device. For example, the optical axis of the crystalline lens and the optical axis L1 of the device substantially coincide by tilting the fixation lamp toward the nose by 4° and to an upper side by 1°. In this case, the control unit 80 may move the fixation lamp 1 by controlling a drive (not shown).

The control unit 80 obtains the curvature radius of the anterior surface or the posterior surface of the crystalline lens from the tomographic images as the plurality of captured cross sections. For example, the control unit 80 obtains the shape of the anterior surface or the posterior surface of the crystalline lens by image processing of the tomographic image, and calculates the curvature radius thereof. For example, the curvature radius may be calculated from a curve of a contour of the anterior surface or the posterior surface of the crystalline lens detected by the edge detection of the tomographic image, as shown in Fig. 5.

For example, in a case where the curvature radius at each scanning angle is obtained, the control unit 80 estimates the position of the major axis or the minor axis of the crystalline lens from the relationship between the scanning angle and the curvature radius.

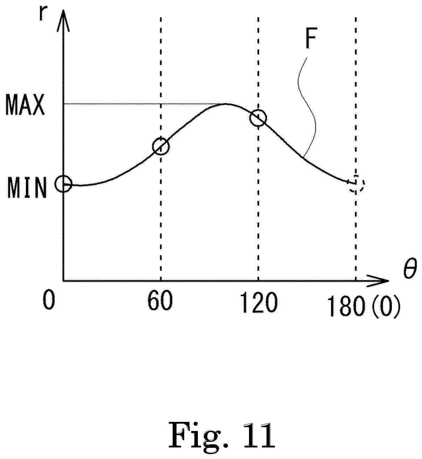

For example, in a case where the crystalline lens is not a perfect circle, it is considered that a section of which a curvature radius is large is positioned in the major axis of the crystalline lens and a section of which a curvature radius is small is positioned in the minor diameter of the crystalline lens. Accordingly, the control unit 80 plots the curvature radius of the crystalline lens anterior surface or posterior surface at each scanning angle on three cross sections, and interpolates the curvature radius corresponding to another angle, as shown in Fig. 11. For example, Lagrange interpolation, spline interpolation, or the like is used. Of course, function approximation may be performed by a least squares method. The control unit 80 obtains the maximum value and the minimum value of a curvature radius r based on the interpolated value or an approximate curve F acquired by the function approximation, and sets a position (angle $\theta$) in which the maximum value is acquired as the major axis of the crystalline lens and a position in which the minimum value is acquired as the minor axis of the crystalline lens. The control unit 80 may calculate an angle of one of the major diameter and the minor axis, and may set an angle acquired by adding 90° to the calculated angle as an angle of the other one. In a case where the curvature radii at the scanning angles are equal to each other, the control unit 80 may determine that the crystalline lens has a circular shape.

<Eyeball Model Display>

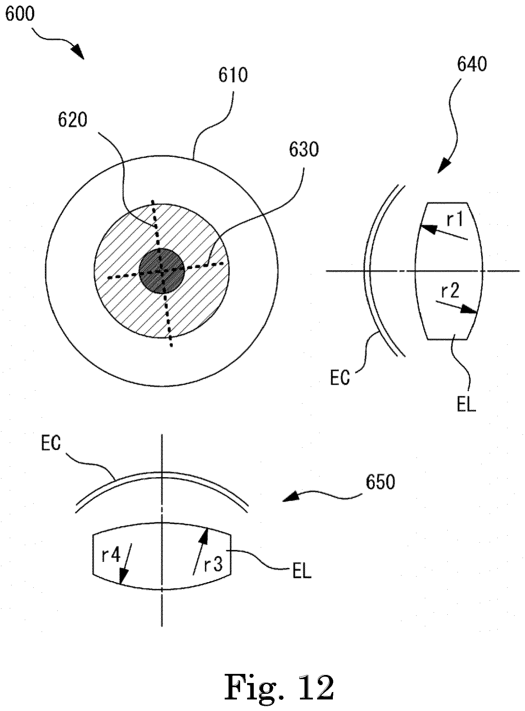

For example, the control unit 80 displays the positions (angles with respect to the rotation axis) of the major axis and the minor axis on the monitor 70. As shown in Fig. 12, the control unit 80 displays the major axis and the minor axis on an eyeball model 610. In the example of Fig. 12, the position of the major axis is displayed by a long dotted line 620, and the position of the minor axis is displayed by a dotted line shorter than the dotted line 630 indicating the major axis are displayed on the eyeball model 610. Accordingly, the examiner can check the tilt of the crystalline lens. The dotted line 620 and the dotted line 630 may be displayed such that a ratio between these lengths is equal to ellipticity of the crystalline lens. Accordingly, the examiner can visualize the ellipsoidal shape of the crystalline lens from the display of the monitor 70.

The control unit 80 may display cross-sectional views of the eyeball model in the positions of the major axis and the minor axis. In the example of Fig. 12, a cross-sectional view 640 in the position of the major axis is displayed beside the eyeball model 61 viewed from the front, and a cross-sectional view 650 in the position of the minor axis is displayed under the eyeball model. For example, the curvature radii of the crystalline lens anterior surface and the crystalline lens posterior surface may be displayed in the respective cross-sectional views. A curvature radius r1 of the crystalline lens anterior surface and a curvature radius r2 of the crystalline lens posterior surface in the position of the major axis and a curvature radius r3 of the crystalline lens anterior surface and a curvature radius r4 of the crystalline lens posterior surface in the position of the minor axis are displayed in Fig. 12. As stated above, it may be easy to visualize the shape of the crystalline lens by representing the curvature radii as numerical values. An image of the actual subject eye may be displayed instead of the aforementioned eyeball model.

<Toric Axis Display>

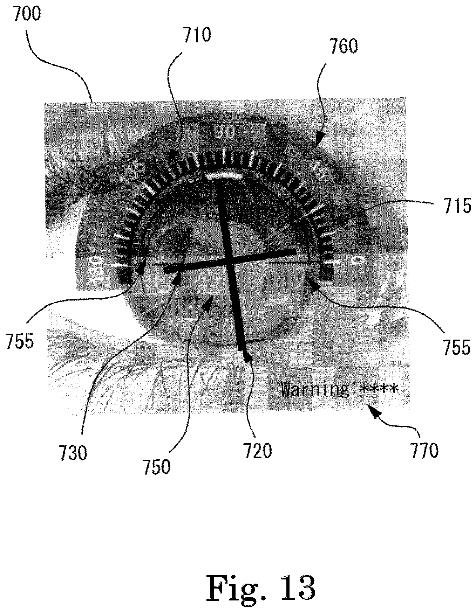

The control unit 80 may display a position of the toric axis of the intraocular lens on the monitor 70. In the example of Fig. 13, the control unit 80 may display a position of a toric axis 710, a position of a loop tip 755 of an intraocular lens 750, and positions of a crystalline lens major axis 720 and a crystalline lens minor axis 730 on an anterior segment front face image of the subject eye. The position of the toric axis 710 may be acquired from another device, or may be calculated based on the measurement result of the OCT device 5, the corneal shape measurement device 300, or the like. In the present example, an astigmatic axis angle of the cornea acquired by the corneal shape measurement device 300 is used as the toric axis. The position of the loop tip 755 is determined by the kind of the intraocular lens, and is obtained from the position of the toric axis. Accordingly, the control unit 80 may store the kind of the intraocular lens to be inserted in the subject eye and the shape thereof in the memory 85 in advance.

The intraocular lens is supported by the capsule of the crystalline lens by using elastic force in a case where the loop of the intraocular lens spreads outwards in an attempt to return to an original shape, but the intraocular lens rotates in a direction in which resistance from the crystalline lens capsule is high, that is, toward the major axis from the minor axis of the crystalline lens. Accordingly, in a case where the loop tip 755 is positioned in the crystalline lens minor axis 730, the control unit 80 may display a warning 770 indicating that there is a possibility that the intraocular lens is to revolve on the monitor 70. For example, the control unit 80 may display the warning 770 in a case where the loop tip 755 is within a range of $\pm 5°$ from the crystalline lens minor axis 730. Through the warning display, the examiner may adjust the axis angle of the intraocular lens at the time of inserting the intraocular lens.

The control unit 80 may calculate an angle difference between the position of the loop and the position of the major axis of the crystalline lens in a case where the toric axis of the intraocular lens is disposed at an ideal axis angle with respect to the astigmatic axis of the eye. In a case where this angle is equal to or greater than a predetermined angle (for example, 10° or more), the control unit 80 may display the warning 770 on the monitor 70.

In a case where it is determined that there is a possibility that the intraocular lens will revolve, the control unit 80 may display information indicating that the intraocular lens shall be shifted in a direction opposite to the revolution direction in advance and then is inserted as the warning 770. For example, in a case where the position of the loop tip 755 is closer to the crystalline lens minor axis 730 than the crystalline lens major axis 720, the control unit 80 may display an instruction to prompt the loop tip to be inserted in the eye by shifting the toric axis by about 5° in a direction in which the loop tip 755 moves away from the major axis on the monitor 70. In a case where it is determined that there is a possibility that the intraocular lens will revolve, the control unit 80 may display information indicating that a toric intraocular lens having a stronger astigmatism correction effect shall be inserted, as the warning 770. For example, in the aforementioned example, in a case where it is determined that an axis shift of 5° is caused, the control unit may display an instruction to prompt the power of the toric intraocular lens is reselected on the monitor 70. Accordingly, it is possible to select an intraocular lens having a power acquired by taking the influence of the misalignment of the toric axis due to the revolution of the intraocular lens into consideration.

As stated above, the present device can calculate the position of the major axis and the minor axis of the crystalline lens, and can easily acquire shape information of the crystalline lens. Accordingly, the examiner can determine the axis angle of the intraocular lens by taking the influence of the revolution of the intraocular lens due to the shape of the crystalline lens into consideration.

The control unit 80 may store the result acquired by calculating the misalignment amount between the position of the crystalline lens major axis or minor axis and the toric axis or the position of the loop, as well as the revolution amount of the intraocular lens after surgery, in the memory 85. In this case, the control unit 80 may estimate a revolution amount of an intraocular lens of another eye (a right eye or a left eye, or an eye of another patient) being examined based on the relationship between the misalignment amount before surgery stored in the memory 85 and an actual revolution amount after surgery.

For example, the control unit 80 stores the misalignment amount between the position of the loop predicted before surgery and the position of the crystalline lens major axis in the memory 85. The control unit 80 stores the actual revolution amount measured after surgery in the memory 85. For example, the actual revolution amount is obtained by calculating the misalignment amount (revolution amount) between the position of the loop predicted before surgery and the position of the loop measured after surgery. For example, the actual position of the loop after surgery is obtained by identifying the shape of the intraocular lens by image analysis of the anterior segment front face image. For example, the control unit 80 may predict a revolution amount of an intraocular lens of the nest patient based on a tendency of the revolution amount of the intraocular lens after surgery with respect to the misalignment amount between the loop before surgery and the crystalline lens major axis accumulated in the memory 85. For example, in a case where there is an increasing tendency of the revolution amount as the misalignment amount is increased, the control unit 80 may predict that the revolution amount becomes large as the misalignment amount becomes large. In a case where there is a decreasing tendency of the revolution amount as the misalignment amount is increased, the control unit 80 may predict that the revolution amount becomes small as the misalignment amount becomes large. As stated above, the control unit 80 may estimate a more appropriate revolution amount by using the past surgery data.

The control unit 80 may predict the revolution amount for each intraocular lens model. Since the tendency of the revolution varies depending on the model of the intraocular lens, the control unit 80 may calculate the revolution amount corresponding to the tendency of each model based on the past data of the revolution amount. Since the tendency of the revolution of the intraocular lens varies depending on a surgical method or habit of the surgeon, the revolution amount corresponding to the tendency of each surgeon may be calculated.

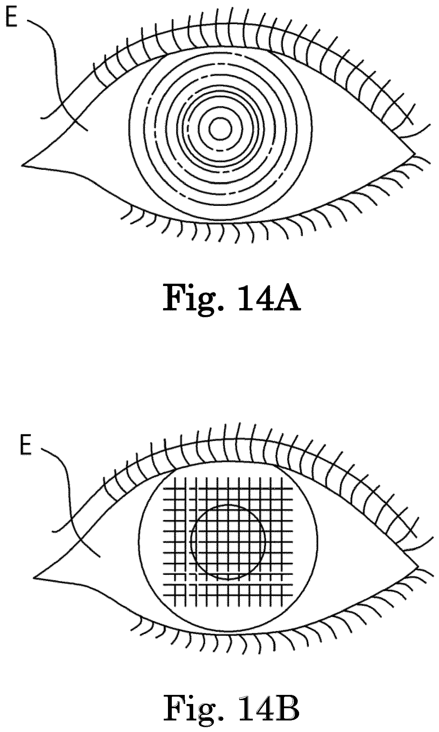

Although it has been described that the scanning method of the OCT optical system 5 is radially performed, circle scanning may be performed in concentric circles, or radial scanning may be performed (see Figs. 14A and 14B). In this case, the control unit 80 may calculate the major axis and the minor axis of the crystalline lens based on three-dimensional shape data of the crystalline lens acquired by the circle scanning or the radial scanning.

The method of calculating the positions of the major axis and the minor axis of the crystalline lens is not limited to the aforementioned example, and there is a method of estimating the equatorial position of the crystalline lens. For example, the equator of the crystalline lens is the maximum diameter section of the crystalline lens. On the anterior segment tomographic image 500 acquired by using the OCT optical system 100 as in the present example, since the measurement light is shielded by the iris, the equator of the crystalline lens is not captured. Accordingly, the control unit estimates a shape of an equator unit based on a shape of a central section of the crystalline lens which is captured. For example, the control unit 80 estimates a point at which a curve (for example, an approximate circle) along the crystalline lens anterior surface and a curve (for example, an approximate circle) along the crystalline lens posterior surface cross each other as the equatorial position. This equatorial position is calculated at each scanning angle, and an ellipsoid passing through these equatorial positions is obtained. In this case, the control unit 80 may display the values of the sizes of the major axis and the minor axis of the obtained ellipsoid as the major axis and the minor axis of the crystalline lens. In this case, the control unit 80 may display the values of the sizes of the major axis and the minor axis of the obtained ellipsoid on the display unit, or may display a length of a graphic image such as the line indicating the major axis or the minor axis and the size thereof.

The control unit 80 may use the curvature of the crystalline lens anterior surface and/or the curvature of the crystalline lens posterior surface at the time of calculating the major axis and the minor axis. Since the positions of the major axis and the minor axis are substantially equal to each other using any curvature of the crystalline lens anterior surface and posterior surface, the control unit 80 compares the calculation results, and checks the validity of the calculation results. For example, in a case where the difference between the calculation results is equal to or greater than a predetermined amount (for example, 5° or more), the control unit 80 may determine that the calculation results are not valid, and may capture the cross section of the subject eye again.

Although it has been described in Fig. 12 or 13 that the major axis and the minor axis of the crystalline lens are displayed by the lines, the figure of the estimated ellipsoid may be displayed. In this case, the examiner can also estimate an ellipsoidal shape and tilt conditions of the major axis and the minor axis of the crystalline lens.

**Reference Signs List**

| | |
|---|---|
| 5 | optical coherence tomography device |
| 10 | ophthalmologic device |
| 30 | anterior segment front face imaging optical system |
| 40 | alignment projection optical system |
| 50 | keratometer projection optical system |
| 70 | monitor |
| 80 | control unit |
| 85 | memory |
| 84 | operation unit |

**Claims**

1. An ophthalmologic device for determining a power of an IOL to be inserted in a subject eye, comprising:

   a parameter acquisition portion that acquires a plurality of eye shape parameters of the subject eye; and
   a calculation control portion that calculates the IOL power,
   wherein the calculation control portion outputs IOL related information with a mathematic model that has been trained by a machine learning algorithm by inputting the plurality of eye shape parameters into the mathematic model.

2. The ophthalmologic device according to claim 1,
   wherein the calculation control portion calculates the IOL power based on a predicted postoperative anterior chamber depth that is output as the IOL related information from the mathematic model.

3. The ophthalmologic device according to claim 1 or 2,
   wherein the mathematic model is trained by using a plurality of training datasets using the plurality of eye shape parameters as input training data and using postoperative anterior chamber depth as output training data.

4. The ophthalmologic device according to any one of claims 1 to 3,
   wherein among the eye shape parameters is a crystalline lens shape parameter.

**5.** The ophthalmologic device according to any one of claims 2 to 4,
wherein the calculation control portion calculates the IOL power by substituting the predicted postoperative anterior chamber depth output by the mathematic model into an IOL power calculation formula.

**6.** The ophthalmologic device according to claim 1,
wherein the calculation control portion outputs selection information for selecting an appropriate formula among a plurality of IOL power calculation formulas to the mathematic model.

**7.** The ophthalmologic device according to claim 5, further comprising:
a presentation portion that presents the selection information output by the mathematic model to a user.

**8.** The ophthalmologic device according to claim 5, further comprising:
a warning portion that warns a user in a case where the user attempts to use an inappropriate formula in the selection information output by the mathematic model.

**9.** The ophthalmologic device according to claim 1,
wherein the calculation control portion outputs the IOL power as the IOL related information from the mathematic model after inputting the plurality of eye shape parameters to the mathematic model.

**10.** The ophthalmologic device according to claim 9,
wherein the calculation control portion outputs the IOL power with the mathematic model by calculating predicted postoperative anterior chamber depth based on the plurality of eye shape parameters, after inputting the eye shape parameters and the predicted postoperative anterior chamber depth to the mathematic model.

**11.** The ophthalmologic device according to claim 9,
wherein the calculation control portion outputs the IOL power from a second mathematic model after inputting the plurality of eye shape parameters to a first mathematic model and inputting a predicted postoperative anterior chamber depth output from the first mathematic model to the second mathematic model different from the first mathematic model.

**12.** The ophthalmologic device according to any one of claims 1 to 11,
wherein the calculation control unit inputs a feature parameter regarding at least any feature of race, gender, and age of a patient, IOL model, and surgeon to the mathematic model in addition to the eye shape parameters.

**13.** The ophthalmologic device according to claim 12,
wherein the feature parameter includes a lens constant to be used in each IOL power calculation formula.

**14.** The ophthalmologic device according to any one of claims 1 to 13,
wherein the calculation control portion outputs a toric IOL power from the mathematic model.

**15.** The ophthalmologic device according to claim 14,
wherein the eye shape parameter includes at least any of corneal anterior surface astigmatism/astigmatic axis, corneal posterior surface astigmatism/astigmatic axis, corneal thickness, anterior chamber depth, postoperative anterior chamber depth, surgically induced astigmatism, pupil diameter, crystalline lens anterior surface astigmatism/astigmatic axis, crystalline lens posterior surface astigmatism/astigmatic axis, incision position, incision width, incision angle, auxiliary port position, auxiliary port number, and auxiliary port width.

**16.** The ophthalmologic device according to claim 15,
wherein the calculation control portion calculates an astigmatic power of the toric IOL based on the crystalline lens anterior surface astigmatism and the crystalline lens posterior surface astigmatism.

**17.** The ophthalmologic device according to any one of claims 1 to 16,
wherein the calculation control portion outputs surgically induced astigmatism from the mathematic model by inputting feature parameters regarding at least any of an incision position, an incision width, an incision angle, an auxiliary port position, an auxiliary port number, auxiliary port width, and a surgeon to the mathematic model.

**18.** The ophthalmologic device according to claim 1,
wherein the calculation control portion outputs a plurality of pieces of the IOL related information including at least any of IOL power and a predicted postoperative anterior chamber depth from the mathematic model by inputting the plurality of eye shape parameters to the mathematic model.

19. The ophthalmologic device according to any one of claims 1 to 18,
wherein the calculation control portion further outputs at least any of a degree of progress of an intermediate transparent ocular opacity and a degree of progress of corneal degeneration from the mathematic model.

20. The ophthalmologic device according to any one of claims 1 to 19,
wherein the eye shape parameter is acquired from a server system connected via a communication network.

21. The ophthalmologic device according to any one of claims 1 to 20, further comprising:
a measurement portion for measuring the eye shape parameters of the subject eye.

22. An IOL power determination program executed in an ophthalmologic device for determining a power of an IOL to be inserted in a subject eye,
wherein the IOL power determination program is executed by a processor of the ophthalmologic device to cause the ophthalmologic device to perform:

a parameter acquisition step of acquiring a plurality of eye shape parameters of the subject eye; and
a calculation step of outputting IOL related information from a mathematic model trained by a machine learning algorithm by inputting the plurality of eye shape parameters to the mathematic model.

23. An ophthalmologic device for determining a power of an intraocular lens to be inserted in a subject eye, comprising:

a cross-sectional imaging portion that captures an anterior segment cross-sectional image of the subject eye; and
a calculation control portion that calculates the power of the intraocular lens,
wherein the calculation control portion analyzes the anterior segment cross-sectional image to acquire a capsule diameter of a crystalline lens of the subject eye, estimates a predicted postoperative anterior chamber depth of the intraocular lens by using the capsule diameter, and calculates the power of the intraocular lens based on the predicted postoperative anterior chamber depth.

24. The ophthalmologic device according to claim 23,
wherein the calculation control portion estimates the predicted postoperative anterior chamber depth based on the capsule diameter and a feature parameter of the intraocular lens.

25. The ophthalmologic device according to claim 24,
wherein the feature parameter includes at least an entire length of the intraocular lens.

26. The ophthalmologic device according to claim 24,
wherein the feature parameter includes at least any of an entire length of the intraocular lens, a thickness of an optical section, an elastic modulus of an optical section, an optical section diameter, an angle of a loop, a thickness of a loop, and an elastic modulus of a loop.

27. The ophthalmologic device according to any one of claims 23 to 26,
wherein the calculation control portion performs correction such that a predicted value of the predicted postoperative anterior chamber depth is corrected to be closer to a posterior capsule as the capsule diameter is smaller than the entire length.

28. The ophthalmologic device according to claim 27,
wherein the calculation control portion uses a correction amount calculated based on the capsule diameter and the feature parameter for calculating the predicted value.

29. The ophthalmologic device according to any one of claims 23 to 28,
wherein the calculation control portion compares the entire length with a size of the capsule diameter, and determines whether or not the intraocular lens is appropriate for the subject eye based on the comparison result.

30. The ophthalmologic device according to claims 23 to 29,
wherein the calculation control portion estimates the predicted postoperative anterior chamber depth based on an anterior chamber depth of a subject eye, an offset distance from a crystalline lens anterior surface to a crystalline lens equator, and a correction amount calculated by using the capsule diameter.

31. The ophthalmologic device according to any one of claims 23 to 30, wherein the calculation control portion assumes that an intersection of a curve along a crystalline lens anterior surface and a curve along a crystalline lens posterior surface on the anterior segment cross-sectional image is an equatorial position, and calculates the capsule diameter of the crystalline lens.

32. An IOL power determination program executed in an ophthalmologic device that determines a power of an intraocular lens to be inserted in a subject eye, wherein the IOL power determination program is executed by a processor of the ophthalmologic device to cause the ophthalmologic device to perform:

a cross-section imaging step of capturing an anterior segment cross-sectional image of the subject eye; a capsule diameter acquisition step of analyzing the anterior segment cross-sectional image captured in the cross-section imaging step to acquire a capsule diameter of a crystalline lens of the subject eye; an estimation step of estimating a predicted postoperative anterior chamber depth of the intraocular lens by using the capsule diameter acquired in the capsule diameter acquisition step; and a calculation step of calculating the power of the intraocular lens based on the predicted postoperative anterior chamber depth estimated in the estimation step.

33. An ophthalmologic device for imaging an anterior segment of a subject eye, comprising:

a tomographic imaging portion that captures an anterior segment tomographic image of the subject eye; and a calculation control portion that calculates at least any position of a major axis and a minor axis of a crystalline lens of the subject eye based on the anterior segment tomographic image.

34. The ophthalmologic device according to claim 33, further comprising: a display control portion that displays at least any position of the major axis and the minor axis of the crystalline lens on a display portion.

35. The ophthalmologic device according to claim 34, wherein the display control portion displays at least any position of the major axis and the minor axis of the crystalline lens and a position of an astigmatic axis of a toric intraocular lens on the display portion.

36. The ophthalmologic device according to claim 34 or 35, wherein the display control portion displays at least any position of the major axis and the minor axis of the crystalline lens and a position of a loop of the toric intraocular lens on the display portion.

37. The ophthalmologic device according to any one of claims 33 to 36, wherein the calculation control portion calculates the misalignment amount between at least any position of the major axis and the minor axis of the crystalline lens and at least any position of the astigmatic axis and the loop.

38. The ophthalmologic device according to any one of claims 33 to 37, further comprising a notification portion, wherein the calculation control portion causes the notification portion to notify that there is a possibility that the toric intraocular lens will revolve, in a case where the misalignment amount between the position of the major axis and the position of the loop exceeds a predetermined amount.

39. The ophthalmologic device according to any one of claims 33 to 37, further comprising a notification portion, wherein the calculation control portion causes the notification portion to notify that there is a possibility that the toric intraocular lens will revolve, in a case where a tip of a loop of the toric intraocular lens is in the position of the minor axis.

40. The ophthalmologic device according to claim 38 or 39, wherein the calculation control portion causes the notification portion to communicate the direction in which the toric intraocular lens revolves.

41. The ophthalmologic device according to any one of claims 38 to 40, wherein the calculation control portion causes the notification portion to give an instruction to shift a tip of the loop in a direction opposite to the revolution direction when inserting the intraocular lens.

42. The ophthalmologic device according to any one of claims 34 to 41, further comprising:

a front imaging portion that captures a front face image of the subject eye,
wherein the display control portion displays the position of at least any of the major axis and the minor axis of the crystalline lens on the front face image in a superimposition manner.

43. The ophthalmologic device according to claim 37,
wherein the calculation control portion stores the misalignment amount calculated before surgery and a revolution amount of the intraocular lens after surgery in a storage portion.

44. The ophthalmologic device according to claim 43,
wherein the calculation control portion predicts a revolution amount of an intraocular lens in another subject eye based on the misalignment amount and the revolution amount after surgery stored in the storage portion.

45. The ophthalmologic device according to claim 44,
wherein the calculation control portion performs prediction on one or both of an intraocular lens model and a surgeon.

**Fig. 1**

Fig. 2

START

S1 — ALIGNMENT

S2 — CAPTURE FRONT IMAGE

S3 — CAPTURE TOMOGRAPHIC IMAGE

S4 — ACQUIRE EYE SHAPE PARAMETER

S5 — INPUT PARAMETER TO MATHEMATIC MODEL

S6 — OUTPUT PREDICTED POSTOPERATIVE ANTERIOR CHAMBER DEPTH

S7 — SUBSTITUTE EACH PARAMETER INTO EXPRESSION

S8 — ACQUIRE IOL POWER

END

Fig. 3

Fig. 4

Fig. 5

Fig. 6

Fig. 7

Fig. 8A

Fig. 8B

Fig. 9

Fig. 10

Fig. 11

600

610

620

630

640

r1

r2

EC

EL

EC

650

r4

r3

EL

Fig. 12

Fig. 13

Fig. 14A

Fig. 14B

## INTERNATIONAL SEARCH REPORT

International application No.

PCT/JP2017/027547

**A.  CLASSIFICATION OF SUBJECT MATTER**

*A61B3/10*(2006.01)i

According to International Patent Classification (IPC) or to both national classification and IPC

**B.  FIELDS SEARCHED**

Minimum documentation searched (classification system followed by classification symbols)

A61B3/10

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2017 |
| Kokai Jitsuyo Shinan Koho | 1971-2017 | Toroku Jitsuyo Shinan Koho | 1994-2017 |

Electronic data base consulted during the international search (name of data base and, where practicable, search terms used)

**C.  DOCUMENTS CONSIDERED TO BE RELEVANT**

| Category* | Citation of document, with indication, where appropriate, of the relevant passages | Relevant to claim No. |
|---|---|---|
| X | FINDL Oliver et al., Analysis of nonlinear systems to estimate intraocular lens position after cataract surgey, Journal of Cataract & Refractive Surgery, Volume 30, Issue 4, 2004.04. 30, page 863-866 | 1-5,9-11,18, 22 |
| Y | | 6-8,12-17, 19-21 |
| Y | JP 2011-206368 A  (Nidek Co., Ltd.), 20 October 2011 (20.10.2011), paragraph [0054] (Family: none) | 6-8,12-17, 19-21 |

☒  Further documents are listed in the continuation of Box C.          ☐  See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| | |
|---|---|
| Date of the actual completion of the international search | Date of mailing of the international search report |
| 09 August 2017 (09.08.17) | 17 October 2017 (17.10.17) |
| Name and mailing address of the ISA/ Japan Patent Office 3-4-3,Kasumigaseki,Chiyoda-ku, Tokyo 100-8915,Japan | Authorized officer Telephone No. |

Form PCT/ISA/210 (second sheet) (January 2015)

**INTERNATIONAL SEARCH REPORT**

| International application No. |
| --- |
| PCT/JP2017/027547 |

C (Continuation).　DOCUMENTS CONSIDERED TO BE RELEVANT

| Category* | Citation of document, with indication, where appropriate, of the relevant passages | Relevant to claim No. |
| --- | --- | --- |
| Y | JP 2003-75785 A (Vision Opt. Co., Ltd.), 12 March 2003 (12.03.2003), paragraphs [0139] to [0140]; fig. 20 & US 2004/0174499 A1 paragraphs [0292] to [0297]; fig. 20 & WO 03/000123 A1　　& CA 2449996 A1 & CN 1520270 A | 12-17,19-21 |
| Y | WO 2015/020731 A2 (NOVARTIS AG), 12 February 2015 (12.02.2015), claims 1 to 2 & JP 2016-531666 A　　　& US 2015/0046094 A1 | 14-17,19-21 |
| Y | JP 2005-288176 A (Nidek Co., Ltd.), 20 October 2005 (20.10.2005), claim 1 & US 2005/0225724 A1 claim 1 | 19-21 |
| A | JP 2007-531559 A (Ophthonix, Inc.), 08 November 2007 (08.11.2007), paragraphs [0023] to [0045]; fig. 1 to 9 & US 2005/0200809 A1 paragraphs [0025] to [0048]; fig. 1 to 9 & WO 2005/079546 A2 | 1-22 |
| A | JP 2013-94410 A (Nidek Co., Ltd.), 20 May 2013 (20.05.2013), paragraphs [0011] to [0071]; fig. 1 to 5 & US 2013/0107208 A1 paragraphs [0021] to [0085]; fig. 1 to 5 & EP 2586361 A1 | 23-32 |
| A | JP 2013-503020 A (PowerVision, Inc.), 31 January 2013 (31.01.2013), claims 1 to 2 & US 2011/0052020 A1 claims 1 to 2 & WO 2011/026068 A2 | 23-32 |
| X Y | JP 2016-29968 A (Canon Inc.), 07 March 2016 (07.03.2016), paragraphs [0008] to [0060]; fig. 1 to 15 (Family: none) | 33-42 43-45 |
| Y | JP 2011-188983 A (Nidek Co., Ltd.), 29 September 2011 (29.09.2011), paragraphs [0010] to [0071]; fig. 1 to 5 (Family: none) | 43-45 |

Form PCT/ISA/210 (continuation of second sheet) (January 2015)

**INTERNATIONAL SEARCH REPORT**

International application No.

PCT/JP2017/027547

C (Continuation).  DOCUMENTS CONSIDERED TO BE RELEVANT

| Category* | Citation of document, with indication, where appropriate, of the relevant passages | Relevant to claim No. |
|---|---|---|
| X | WO 2013/187361 A1 (The Kitasato Institute), 19 December 2013 (19.12.2013), paragraphs [0031] to [0079]; fig. 1 to 13 & US 2015/0092162 A1 paragraphs [0050] to [0100]; fig. 1 to 13 | 33 |

Form PCT/ISA/210 (continuation of second sheet) (January 2015)

**INTERNATIONAL SEARCH REPORT**

| International application No. |
| --- |
| PCT/JP2017/027547 |

---

**Box No. II    Observations where certain claims were found unsearchable (Continuation of item 2 of first sheet)**

This international search report has not been established in respect of certain claims under Article 17(2)(a) for the following reasons:

1. ☐ Claims Nos.:
   because they relate to subject matter not required to be searched by this Authority, namely:

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

---

**Box No. III    Observations where unity of invention is lacking (Continuation of item 3 of first sheet)**

This International Searching Authority found multiple inventions in this international application, as follows:
See extra sheet.

1. ☒ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**
☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☒ No protest accompanied the payment of additional search fees.

Form PCT/ISA/210 (continuation of first sheet (2)) (January 2015)

**INTERNATIONAL SEARCH REPORT**

International application No.

PCT/JP2017/027547

Continuation of Box No.III of continuation of first sheet(2)

The invention of claim 1 has no special technical feature, since the invention lacks novelty in the light of the document 1 (FINDL Oliver et al., Analysis of nonlinear systems to estimate intraocular lens position after cataract surgery, Journal of Cataract & Refractive Surgery, Volume 30, Issue 4, 2004.04.30, page 863-866).

Claims are respectively classified into three inventions each of which has a special technical feature indicated below.

Meanwhile, claim 1 having no special technical feature is classified into Invention 1.

(Invention 1) claims 1-22

An ophthalmological device, wherein "an arithmetic and control means inputs the plurality of eye shape parameters to a mathematical model trained using a machine learning algorithm to thereby output IOL-related information from the mathematical model".

(Invention 2) claims 23-32

An ophthalmological device, wherein "an arithmetic and control means acquires a capsule diameter of a crystalline lens of the eye to be examined by analyzing the anterior ocular segment sectional image, estimates a predicted postoperative anterior chamber depth of the intraocular lens using the capsule diameter, and calculates the power of the intraocular lens on the basis of the predicted postoperative anterior chamber depth".

Claims 23-32 are not relevant to inventions which involve all of the matters to define the invention in claim 1 and which have a same category.

Further, as a result of the search which has been carried out with respect to claims classified into Invention 1, claims 23-32 are not relevant to inventions on which it is substantially possible to carry out a search without an additional prior-art search and judgment, and there is no other reason for that it can be considered that it is efficient to carry out a search on these claims together with claims 1-22, and consequently, it is impossible to classify claims 23-32 into Invention 1.

(Invention 3) claims 33-45

An ophthalmological device provided with "an arithmetic and control means for controlling the position of a long diameter and/or a short diameter of a crystalline lens of the eye to be examined on the basis of the anterior ocular segment sectional image".

Claims 33-45 are not relevant to inventions which involve all of the matters to define the invention in claim 1 or 23 and which have a same category.

Further, as a result of the search which has been carried out with respect to claims classified into Invention 1 or 2, claims 33-45 are not relevant to inventions on which it is substantially possible to carry out a search without an additional prior-art search and judgment, and there is no other reason for that it can be considered that it is efficient to carry out a search on these claims together with claims 1-22 or claims 23-32, and consequently, it is impossible to classify claims 33-45 into Invention 1 or 2.

Form PCT/ISA/210 (extra sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2011206368 A
- JP 2013094410 A